(19) Europäisches Patentamt / European Patent Office / Office européen des brevets

(11) **EP 3 494 872 A1**

(12) **DEMANDE DE BREVET EUROPEEN**

(43) Date de publication:
**12.06.2019 Bulletin 2019/24**

(51) Int Cl.:
***A61B 5/00*** *(2006.01)*

(21) Numéro de dépôt: **18210290.5**

(22) Date de dépôt: **05.12.2018**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité: **07.12.2017 FR 1771325**
**05.05.2018 US 201862667515 P**
**01.11.2018 US 201816178365**

(71) Demandeur: **Ellcie Healthy**
**06270 Villeneuve Loubet (FR)**

(72) Inventeur: **PEYRARD, Philippe**
**06600 Antibes (FR)**

(74) Mandataire: **Ipside**
**29, rue de Lisbonne**
**75008 Paris (FR)**

(54) **SYSTÈME PERSONNEL D'ALERTE PHYSIOLOGIQUE**

(57) L'invention concerne un système comprenant une paire de lunettes comprenant des branches articulées et comprenant une pluralité de capteurs et des moyens d'alerte, comprenant au moins 2 capteurs parmi :
- un accéléromètre triaxial (251) ;
- un émetteur et un récepteur de lumière infrarouge (151, 152);
- un capteur barométrique (252) ;
- une paire d'électrodes (161, 162) alimentées en courant alternatif et des moyens pour mesurer une impédance entre ces deux électrodes ;
lesdits capteurs étant installés sur les branches ou dans la monture des verres de la paire de lunettes et étant connectés à une unité de traitement et de calcul comprenant :
- un microprocesseur et des moyens de mémoire ;
ladite unité de traitement et de calcul comprenant un programme informatique pour l'analyse des données issues des capteurs, et le déclenchement des moyens d'alerte en fonction de l'analyse de ces données.

**Fig. 2**

**Description**

La présente invention est un perfectionnement du dispositif décrit dans la demande de brevet EP17206054 déposée le 7 décembre 2017 ayant pour titre système personnel pour la détection d'une situation à risque et d'alerte.

L'invention appartient au domaine des dispositifs portables aptes à mesurer des données physiologiques d'un individu et de détecter par l'intermédiaire de ces mesure une situation de risque ou de danger relative à la physiologie dudit individu.

De telles situations à risque interviennent, par exemple, en cas de baisse de la vigilance, suite à un endormissement, une perte de connaissance, un état de déshydratation ou encore en cas de chute, sans que ces exemples ne soient limitatifs.

Une telle situation à risque est susceptible d'avoir des conséquences dramatiques, par exemple lorsque la personne qui la subit conduit un véhicule ou une machine, le risque s'étendant aux occupants du véhicule ou aux installations et personnes à proximité de la machine.

Une chute, même légère, est potentiellement un danger vital lorsqu'elle touche une personne âgée ou a mobilité réduite.

Une personne, particulièrement une personne âgée, peut atteindre un état de déshydratation avancé sans ressentir la soif. Un tel état conduit à une baisse de vigilance, une perte de connaissance voire à des symptômes plus sévères.

Les risques engendrés par ces situations sont considérablement réduits si des mesures appropriées sont exécutées à temps.

Ainsi, à titre d'exemple, le conducteur d'un véhicule surestime facilement son état de vigilance, au point de se faire surprendre par un réel assoupissement. Une simple alarme à son intention ou à l'intention des passagers du véhicule permet de lui faire prendre conscience de son état et de l'encourager à stopper la conduite.

Les conséquences d'une chute, même lourde, sont réduites si une assistance est apportée à la personne dans un délai court. D'autres cas comparables concernent, par exemple, des malaises.

Les personnes pratiquant des activités de plein air telles que la randonnée ou le cyclisme, ou une personne âgée dans des conditions météorologiques de fortes chaleur, un conducteur de véhicule, sont soumis à des risques de déshydratation.

La déshydratation peut entraîner des troubles fonctionnels, empêchant par exemple la personne âgée de boire ou de communiquer son besoin en liquide, provoquer de la confusion mentale, une mauvaise concentration, une altération de la mémoire et une difficulté générale à accomplir des tâches, augmentant ainsi le risque d'accident pour le conducteur, ou le risque de se perdre pour le randonneur.

A titre d'exemple, il est considéré que 21 % des accidents mortels de la circulation ont pour cause initiale une baisse significative de vigilance ou un endormissement du conducteur.

Les chutes sont la première cause de mortalité des personnes de plus de 65 ans. En France, 12 000 personnes par an décèdent à la suite d'une chute. Hormis les cas de décès, les chutes engendrent chez les personnes qui en sont victimes des pertes d'autonomie et des pertes de confiance en soi qui ont également des conséquences importantes.

Les études montrent que, dans le cas d'une personne âgée, plus le temps passé au sol après une chute est long et plus les conséquences sont graves.

Les troubles de la vigilance sont également des causes de chute, ils sont causés par une fatigue extrême, ou plus couramment par la prise de médicaments ou l'absorption d'alcool.

Ainsi, la détection d'une perte de vigilance et l'avertissement de la personne qui en est l'objet ou d'un de ses proches, permet également dans certaines circonstances de prévenir une chute.

La déshydratation, la perte de vigilance et la chute sont ainsi liées dans de nombreux cas. La déshydratation peut provoquer des vertiges et une perte de vigilance, voire une perte de conscience conduisant finalement à une chute.

L'hydratation étant influencée par la consommation d'alcool et de drogues, une détection précoce de la déshydratation permet dans certains cas prévenir un risque supplémentaire de perte de vigilance.

Une détection précoce d'une baisse de vigilance peut empêcher une chute. La gravité d'une chute et la capacité de la victime à se remettre d'une chute peuvent être évaluées en mesurant la vigilance de la personne après la chute.

Des mesures physiologiques effectuées en laboratoire sur des groupes d'individus permettent de détecter statistiquement des signes de baisse de vigilance, d'endormissement, de chute, de perte de connaissance ou de déshydratation. Ces expériences utilisent de multiples capteurs qui ne peuvent être portés par un individu qu'en condition de laboratoire.

Lorsque les résultats de ces expériences sont utilisées à des fins de détection personnelle, la qualité de la détection baisse, pour diverses raisons, parmi lesquelles :

- il est difficile d'intégrer les capteurs de mesure adaptés, en nombre et en fiabilité de mesure, dans un dispositif portable ;
- le dispositif portable est inesthétique, inconfortable, trop intrusif, ou trop perçu par l'individu et ses proches comme un dispositif de surveillance, de sorte que l'individu ne le porte pas ;
- la fiabilité de la détection n'est pas satisfaisante, du fait du nombre réduit de capteurs et du fait qu'elle est basée sur des données statistiques non adaptées à l'individu lui-même et à son mode de vie, et conduit à la génération de fausses alertes, de sorte que la personne perd sa confiance dans le dispositif et ne le porte plus ;

- la détection intervient trop tardivement, à titre d'exemple les systèmes détectant un endormissement par la fermeture continue des yeux, pendant une seconde ou plus, ou par la chute de la tête, détectent en fait un état avancé de perte de vigilance. Si cette détection, et les actions éventuelles qu'elle déclenche présentent une utilité certaine, une voiture lancée à 130 km/h parcourt 36 mètres en une seconde, de quoi réduire considérablement l'efficacité de toute manoeuvre effectuée par le conducteur ainsi réveillé dans une situation critique ;

- les système de détection utilise généralement des tests en cascade où le résultat d'un premier test conditionne la mise en oeuvre d'un second test, etc... la fiabilité étant médiocre elle ne fait qu'empirer d'un test à l'autre et génère des faux positifs ou des faux négatifs ;

- l'autonomie des systèmes individuels est réduite du fait de la consommation électrique des nombreux composants et de la puissance de calcul nécessaire.

L'invention vise à résoudre les inconvénients de l'art antérieur en proposant un système reposant sur un support de capteur esthétique, autonome et léger, spécifiquement adapté à son utilisateur.

Le système objet de l'invention utilise un nombre limité de capteurs, de sorte à limiter la consommation électrique pour la détection de 3 événements types, susceptible dans certaines circonstances de s'enchaîner :

- une baisse de vigilance ;
- un état de déshydratation ;
- une chute et l'état de la condition de la personne après la chute.

Bien que le système objet de l'invention puisse être spécifiquement utilisé par un individu à risque, tel qu'une personne âgée, ce système est adapté à tout individu, y compris une personne en parfaite santé.

Le système objet de l'invention détecte une situation à risque dès son initiation, y compris dans des cas où le sujet n'en a pas encore conscience, et jusqu'à des cas avancés et évalue la sévérité de la situation, générant différents niveaux d'alarme selon cette sévérité.

À cette fin l'invention concerne un système comprenant une paire de lunettes comprenant des branches articulées et comprenant une pluralité de capteurs et des moyens d'alerte, comprenant au moins 2 capteurs parmi :

- un accéléromètre triaxial ;
- un émetteur et un récepteur de lumière infrarouge ;
- un capteur barométrique ;

- une paire d'électrodes alimentées en courant alternatif et des moyens pour mesurer une impédance entre ces deux électrodes ;

lesdits capteurs étant installés sur les branches ou dans la monture des verres de la paire de lunettes et étant connectés à une unité de traitement et de calcul comprenant :

- un microprocesseur et des moyens de mémoire ;

ladite unité de traitement et de calcul comprenant un programme informatique pour l'analyse des données issues des capteurs, et le déclenchement des moyens d'alerte en fonction de l'analyse de ces données.

Les capteurs, ainsi disposés au niveau de la tête de l'individu portant la paire de lunettes du système objet de l'invention, sont nécessaires et suffisants pour détecter, selon la combinaison de capteurs utilisée :

- une perte de vigilance ;
- une chute, dure ou molle ;
- un état de déshysdratation ;

et le cas échéant les conséquences suivantes, notamment une chute dure ou molle suivant une perte de vigilance, une perte de vigilance ou une chute suivant un état de déshydratation, l'état de vigilance après la chute, en discriminant les faux positifs et les faux négatifs des véritables alertes par un traitement approprié, également objet de l'invention, des informations délivrées par lesdits capteurs.

Ainsi, selon des modes de réalisation, 3 ou 4 des capteurs cités sont utilisés selon l'éventail de situations à risque dont la détection est visée.

L'utilisation d'un nombre réduit de capteurs pour la détection d'une situation complexe par un traitement approprié des signaux délivrés, permet de réduire la consommation électrique et d'assurer une large autonomie de fonctionnement, tout en conservant un poids réduit pour le confort d'utilisation.

L'installation desdits capteurs dans une paire de lunettes classique, à branches repliables, permet à l'individu de porter le dispositif objet de l'invention de manière discrète et esthétique.

À l'avantage d'autres systèmes personnels de détection tels que des bracelets ou des médaillons, l'installation des capteurs dans une paire de lunettes permet d'assurer un port quasi continu des capteurs par leur utilisateur dans ses heures d'activité, habitué à porter sa paire de lunettes dès le réveil.

Dans tout le texte le terme « ou » doit être interprété comme inclusif (et/ou).

L'invention est avantageusement mise en oeuvre selon les modes de réalisation et les variantes exposés ci-après, lesquels sont à considérer individuellement ou selon toute combinaison techniquement opérante.

Pour assurer et évaluer de manière fiable une situation à risque à partir d'un nombre réduit de capteurs, pour chaque situation à risque dont la détection et l'évaluation sont visées, l'unité de calcul du système objet de l'invention évalue cette situation à partir d'un indice composite.

Ainsi une situation à risque et son niveau de sévérité sont détectés et évalués par le programme informatique à partir d'un premier paramètre issu d'un motif spécifique détecté dans le signal émis par au moins un des capteurs, et un second paramètre issu d'un autre motif spécifique détecté dans le signal émis par au moins un des capteurs, les deux paramètres étant combinés dans un indice composite dont le niveau définit le degré de sévérité de la situation et le déclenchement d'un niveau d'alarme.

Selon des exemples de réalisation les deux paramètres sont calculés à partir du signal émis par un même capteur mais en y appliquant un traitement différent ou en y recherchant un motif différent, ou sont calculés à partir des signaux issus de deux capteurs différents.

Bien entendu l'indice composite est calculé, selon certains modes de réalisation, à partir de plus de deux paramètres.

Selon un mode de réalisation avantageux, le calcul de l'indice composite met en oeuvre au moins deux paramètres dans lesquels le premier paramètre est spécifiquement adapté à l'utilisateur du dispositif et le deuxième paramètre est indicatif du risque quelque soit l'utilisateur. Ainsi la mesure et l'évaluation du premier paramètre font l'objet d'un calibrage et permettent une détection précoce de la situation à risque, alors que la mesure et l'évaluation du deuxième paramètre relèvent de données statistiques et permettent de détecter une situation à risque avérée.

Selon un mode de réalisation particulier, l'unité de traitement et de calcul est également portée par la paire de lunettes objet de l'invention. Ainsi, le dispositif est autonome et ne nécessite pas d'accessoire supplémentaire pour fonctionner.

Selon un autre mode de réalisation, compatible avec le précédent, la paire de lunettes comporte des moyens de connexion sans fil aptes à échanger des informations avec une unité de réception distante. Ce mode de réalisation permet ainsi de déplacer tout ou partie des moyens de traitement et de calcul ainsi que les moyens d'alerte vers un objet indépendant. La paire de lunettes du système objet de l'invention est alors dite connectée.

Selon un exemple de réalisation l'unité distante est un téléphone intelligent. Ainsi, outre la puissance de calcul supplémentaire procurée par les moyens propres du téléphone intelligent, celui-ci est en mesure de communiquer via différents réseaux vers des serveurs ou destinataires distants, que ce soit dans le but d'échanger avec ceux-ci des données mesurées ou de déclencher des alertes et des actions appropriées à une situation donnée.

Avantageusement, l'unité de traitement et de calcul du système objet de l'invention comprend des moyens de connexion à internet et ledit système comprend un serveur central apte à échanger des informations avec ladite unité de traitement et de calcul. Ainsi, les données collectées par le serveur central permettent, par un traitement appropriées de ces données, de faire évoluer les algorithmes de traitement et, par communication entre le serveur central et l'unité de traitement, de mettre à jour le programme informatique de l'unité de traitement et de calcul.

Selon un mode de réalisation, les verres des lunettes sont montés dans des cercles séparés par un pont, l'émetteur et le récepteur infrarouge étant positionnés sur le bord des cercles de sorte à être dirigés vers l'oeil, l'émetteur étant positionné en partie basse de la partie du cercle distale du pont, et le récepteur sur la partie haute proche de la charnière de cette même partie. Ce montage permet de bénéficier de la détection la plus fiable des clignements d'oeil et de fermeture de paupière quelque soit la direction du regard. Avantageusement, ce même dispositif est reproduit sur chacun des cercles de maintien des verres.

Avantageusement, la paire de lunettes comprend une carte électronique logée dans chacune des branches et une liaison filaire entre chaque carte électronique, ladite liaison filaire s'étendant sur la partie supérieure des cercles et le pont. Ainsi la partie inférieure et le montant intérieur des cercles sont libres de tout dispositif électronique ce qui permet d'y monter des verres selon les techniques classiques des opticiens.

Avantageusement, les branches de la paire de lunettes comprennent une partie postérieure connectée mécaniquement à la partie antérieure desdites branches et dépourvue de moyens électroniques pour être adaptée à la morphologie de l'individu.

Avantageusement, les charnières des branches de la paire de lunettes du système objet de l'invention comportent un passage pour la liaison filaire.

Avantageusement, l'accéléromètre triaxial est placé sensiblement au milieu d'une branche. Ainsi, il est positionné sensiblement centré par rapport à la tête du porteur et permet une détection plus fiable de ses mouvements.

Avantageusement, la paire de lunettes comprend une batterie d'alimentation montée dans une des branches, et les cartes électroniques, l'unité de calcul et de traitement, la batterie et les capteurs sont positionnés en partie antérieure des lunettes. La partie antérieure s'étend entre la monture des verres et la partie des branches se positionnant devant les oreilles lors du port des lunettes. Cette configuration permet de conserver de la flexibilité dans l'adaptation des parties postérieures des branches, avec ou sans cambre, à la morphologie de l'utilisateur et ainsi d'assurer un port confortable.

L'invention est exposée ci-après selon ses modes de réalisation préférés, nullement limitatifs, et en référence aux figures 1 à 14, dans lesquelles :

- la figure 1 est une vue en perspective d'un exemple de réalisation de la paire de lunettes du système objet de l'invention ;
- la figure 2 montre selon une vue en perspective, un exemple de réalisation de l'électronique comprise à l'intérieur de la monture des lunettes du système objet de l'invention ;
- la figure 3 représente selon une vue partielle en perspective et en éclaté, un exemple de réalisation de la charnière des branches des lunettes du système objet de l'invention ;
- la figure 4 représente schématiquement un exemple de réalisation de l'organisation du système objet de l'invention dans sa version dite connectée ;
- la figure 5 est un organigramme d'un exemple de traitement du signal conduisant à la génération d'une alarme ;
- la figure 6 montre, selon une vue en perspective, le fonctionnement de l'émetteur et du récepteur infrarouge du système objet de l'invention, figure 6A oeil ouvert et figure 6B oeil fermé ;
- la figure 7 représente un relevé oculométrique issu du récepteur infrarouge du système objet de l'invention ;
- la figure 8 illustre l'évolution temporelle de l'intensité du signal issu du capteur infrarouge et de sa dérivée pendant un clignement d'oeil spontané ;
- la figure 9 représente l'évolution du paramètre AVR en fonction du temps, figure 9A chez un sujet alerte, et figure 9B chez un sujet en baisse de vigilance ;
- la figure 10 montre un exemple de l'évolution de l'accélération mesurée selon la direction de la pesanteur lors d'une micro-chute de la tête ;
- la figure 11, est un organigramme d'un exemple du procédé de traitement des signaux et de déclenchement d'alarme selon l'invention ;
- la figure 12 montre l'évolution du signal perçu par le récepteur infrarouge lors du port et du retrait des lunettes du système objet de l'invention, figure 12A en signal brut, et figure 12B, après filtrage et après dérivation ;
- la figure 13 illustre un exemple d'évolution des signaux lors d'un événement de chute, figure 13A sur le signal d'accélérométrie, figure 13B sur le signal du capteur barométrique ;
- la figure 14 montre un exemple de l'évolution de l'accélération dans un plan perpendiculaire à la gravité au cours d'un événement de chute molle ;

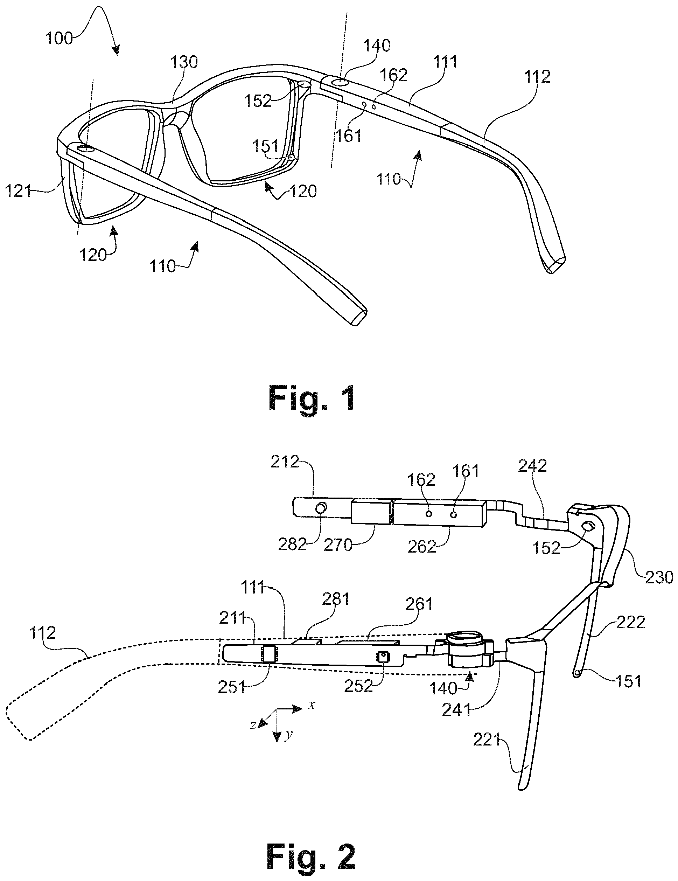

Figure 1, selon un exemple de réalisation, les capteurs du système objet de l'invention sont supportés par une paire de lunettes (100), laquelle paire de lunettes comprend deux branches (110) articulées, deux cercles (120) servant de monture à des verres, correcteurs ou non, lesdits cercles (120) étant connectés par un pont (130) apte à reposer sur le nez de l'utilisateur. Selon cet exemple de réalisation, les branches comprennent deux parties. Une première partie (111), dite antérieure, s'étend depuis la charnière (140) de la branche (110) sur environ la moitié de la longueur de ladite branche. La deuxième partie (112) dite postérieure de la branche est connectée à la première partie (111) par exemple par clippage. Cette deuxième partie est notamment destinée à reposer sur l'oreille de l'utilisateur, et comprend ou non un cambre selon différents styles de lunettes.

Selon cet exemple de réalisation, la partie antérieure de la branche enferme des modules électroniques, alors que la deuxième partie (112) ne comprend aucune électronique. Ainsi, cette deuxième partie est adaptable à la morphologie de l'utilisateur comme toute paire de lunettes classique, en utilisant une deuxième partie (112) plus ou moins longue, ou même en la déformant par chauffage.

De manière similaire, selon un mode de réalisation, la partie extérieure (121) des cercles, s'étendant sensiblement entre la charnière et la base des cercles, supporte des capteurs, notamment un émetteur (151) et un récepteur (152) de lumière infrarouge. La partie inférieure et la partie intérieure des cercles (120), jusqu'au pont (130), est exempte de toute électronique de sorte à faciliter le montage de tout type de verre.

Selon cet exemple de réalisation, les cercles sont en matière plastique et entourent le verre. Le verre est par exemple monté par le chauffage de la partie inférieure des cercles et de leur raccordement par rapport au pont.

La configuration des lunettes du système objet de l'invention permet cependant l'utilisation d'autres types de cercles entre la partie extérieure (121) de ceux-ci et le pont (130). Selon cet exemple de réalisation les plaquettes sont intégrées aux cercles et au pont. Cependant la configuration de la lunette, de la même manière qu'elle permet le montage d'autres types de cercles, permet aussi le montage de plaquettes articulées, lesquelles sont alors ajustables de la même manière que pour une paire de lunettes conventionnelle.

Ainsi les lunettes du système objet de l'invention s'adaptent comme une paire de lunettes conventionnelle à la morphologie de l'utilisateur pour un port, un confort de port et une stabilité optimaux. Les lunettes sont ainsi adaptées à tout type de verre, correcteur ou non, à simple, double foyer ou progressif, ou même sans aucune correction. Elles permettent en outre différentes variantes de style pour adapter leur esthétique aux goûts des utilisateurs.

Le montage des verres et les réglages mécaniques de la paire de lunettes sont préférentiellement réalisés par un professionnel, par exemple un opticien, selon des techniques maîtrisées, car identiques aux techniques utilisées sur les simples lunettes de vue.

Les modules électroniques sont répartis entre les parties antérieures (111) des branches droite et gauche, et sont reliés par un bus souple cheminant dans les parties supérieures des cercles et le pont (130).

Figure 2, selon un exemple de réalisation, la paire de lunettes du système objet de l'invention comprend plusieurs cartes électroniques (211, 212, 221, 222) comportant des circuits imprimés, sur lesquels sont soudés ou encliquetés, notamment, les différents capteurs, les moyens d'acquisition et de calcul ainsi que les moyens de transmission de données.

Lesdites cartes électroniques sont, selon cet exemple de réalisation, logées à l'intérieur de la partie antérieure des branches et à l'intérieure de la partie extérieure des cercles.

Lesdites parties des branches et des cercles sont par exemple constituées d'une matière plastique telle qu'un polyamide ou une acétate ou encore un matériau composite comprenant une matrice thermodurcissable ou thermoplastique renforcée par une charge fibreuse par exemple de verre, de carbone, de lin ou de bambou pour plus de légèreté et de solidité.

Ces enveloppes procurent une protection mécanique et vis-à-vis des conditions climatiques de l'électronique, et sont déclinables en différents aspects et différentes couleurs.

Les cartes électroniques (211, 212, 221, 222) sont connectées entre elles par des bus souples (241, 242, 230). Comprenant un bus central (230) reliant le côté droit et le côté gauche de la paire de lunettes et cheminant à l'intérieur de la partie supérieure des cercles et le pont, et des bus latéraux (241, 242) reliant les cartes (211, 212) comprises dans les parties antérieures des branches et les cartes (221, 222) comprises dans les parties extérieures des cercles.

Les bus latéraux (241, 242) cheminent à travers les charnières (140) des branches, lesquelles charnières sont spécialement conçues à cette fin.

Ainsi, les fonctions de mesure, de traitement du signal, de calcul, de transmission de données et d'alimentation électrique sont réparties essentiellement entre les deux branches, de sorte à répartir de manière sensiblement équivalente le poids entre les deux côtés de la monture.

Les capteurs utilisés sont des capteurs ultraminiaturisés aussi dénommés *« MEMS »* acronyme anglosaxon de «*Micro-Electro-Mechanical-Systems*».

Le nombre de capteurs utilisés dépend des types d'événements dont la détection et l'évaluation sont visés. Selon des exemples de réalisation, seuls sont montés une partie des capteurs ci-après, et dans ce cas la paire de lunettes et le système objet de l'invention sont destinés à la détection d'une ou deux situations à risques définies, ou tous les capteurs décrits sont initialement montés dans la paire de lunettes, ce qui offre la possibilité de détecter potentiellement toutes les situations à risque identifiées, ou une partie seulement, le système pouvant évoluer en activant ou non les modules électroniques correspondant.

Selon un exemple de réalisation, la paire de lunettes comprend un émetteur (151) et un récepteur (152) de lumière infrarouge, placés sur la carte électronique (222) comprise dans la partie extérieure d'un cercle. Cet émetteur et ce récepteur sont dirigés vers l'oeil de l'utilisateur.

Le couple émetteur-récepteur infrarouge est utilisé pour mesurer les mouvement palpébraux dans le but de déterminer la vigilance du sujet portant la paire de lunettes mais également pour mesurer l'état d'hydratation dudit sujet.

Selon un mode de réalisation, le même dispositif est reproduit dans le cercle extérieur gauche et dans le cercle extérieur droit. Le doublement du dispositif permet d'obtenir des mesures sur chaque oeil afin d'examiner la cohérence des signaux obtenus, et de n'utiliser les signaux issus que de l'un ou l'autre couple émetteur-récepteur, dans le cas de défaillance de l'un de ces couples.

Selon un mode de réalisation, combiné ou non aux précédents, un accéléromètre triaxial (251) est placé sur une des cartes électroniques comprises dans les branches, sur la carte électronique (211) de la branche droite selon l'exemple de réalisation représenté figure 2.

Bien qu'un accéléromètre simple, mesurant l'accélération selon une seule direction ($y$) puissent convenir pour plusieurs cas de détection, l'accéléromètre est avantageusement de type triaxial et mesure les accélérations selon trois axes ($x, y, z$). Selon cet exemple, ledit accéléromètre triaxial est monté de sorte que l'accélération de la pesanteur, soit orientée selon l'axe $y$ positif lors du port de la paire de lunettes par l'utilisateur, sans que cette disposition ne soit limitative.

Selon un autre mode de réalisation, le capteur d'accélérométrie est compris dans un capteur MEMS dit inertiel, comprenant un accéléromètre triaxial et un capteur gyroscopique.

Selon encore un autre mode de réalisation, l'accéléromètre est compris dans un capteur MEMS comprenant, un accéléromètre triaxial, un capteur gyroscopique et un compas magnétique intégrés.

Avantageusement, le capteur comprenant l'accéléromètre comprend un sonde de température intégrée, permettant de corriger le signal en gain et en linéarité en fonction de la température du capteur.

Sans que ces caractéristiques ne soient limitatives, l'accéléromètre utilisé dans le système objet de l'invention a une amplitude de mesure de $\pm$ 6g ($\pm$ 58,86 ms$^{-2}$) sur chaque axe.

Comme pour l'émetteur et le récepteur de lumière infrarouge, selon un mode de réalisation, un accéléromètre triaxial est également monté sur la carte électronique (212) de la branche gauche de la paire de lunettes. Ainsi la combinaison des signaux des deux accéléromètres permet de calculer des mouvements de rotation de la tête et de différencier ceux-ci des mouvements de l'ensemble du corps. Le second accéléromètre est ainsi placé de manière symétrique au premier sur l'autre branche.

Les mouvements de la tête, flexion-extension (mouvement du signe « oui »), de rotation axiale (mouvement du signe « non »), ou d'inclinaison latérale, se traduisent notamment par des accélérations opposées selon les axes des deux accéléromètres. Ainsi, par exemple, en se référant au système d'axe (*x, y, z*) de la figure 2, une inclinaison latérale se traduit par des sens opposés d'accélération selon les axes *y* et *z* sur les deux accéléromètres. Une rotation se traduit par des sens opposés d'accélération selon les axes *x* et *z*. La combinaison de ces informations avec les informations issues du capteur gyroscopique et du compas magnétique, dans un mode de réalisation utilisant ce type de capteur, permet de détecter des changements de posture complexes.

Un capteur barométrique (252) est installé, ici sur la carte électronique (211) de la branche droite, mais alternativement sur la carte électronique (212) de la branche gauche. Un tel capteur MEMS est couramment apte à détecter une variation de pression de l'ordre de 6 Pa, ce qui correspond à une variation d'altitude de 50 cm environ. Ainsi, le traitement du signal d'un tel capteur permet par exemple de détecter le passage d'une position debout à une position assise ou couchée et vice-versa, du porteur de la paire de lunettes du système objet de l'invention.

Selon un exemple de réalisation, un couple d'électrodes (161, 162) est placé sur une des branches des lunettes, ici la branche droite, ces électrodes venant en contact avec la peau du porteur dans une zone voisine des tempes.

Lesdites électrodes sont ici représentées juxtaposées et de forme circulaire, mais, selon des variantes, sont disposées superposées et sous formes de bandes.

Lesdites électrodes sont alimentées par un courant alternatif en basse tension (inférieure à 5 volts) et à haute fréquence comprise entre 1 kHz et 100 KHz, préférentiellement autour de 50 KHz.

Lorsque le circuit électrique compris entre les deux électrodes est fermé par l'intermédiaire de la peau avec laquelle elles sont en contact, des moyens de mesure permettent de mesurer l'impédance dudit circuit électrique entre les deux électrodes.

Selon la fréquence et la tension du courant d'alimentation, cette impédance est fonction de la résistance et de la capacitance du circuit ainsi créé. Pour un sujet correctement hydraté, la résistance est typiquement comprise entre 200 et 500 Ohms, typiquement autour de 300 Ohms, la capacitance du circuit se situant entre 80 nF et 100 nF.

La variation d'impédance est, selon un exemple de réalisation, mesurée par la tension entre les deux électrodes (161, 162) une chute de tension de l'ordre de 10 % par rapport au cas nominal d'un sujet bien hydraté correspond à une situation de déshydratation.

Comme précédemment évoqué pour les autres capteurs, la fiabilité de détection est améliorée en doublant ce dispositif, par exemple sur l'autre branche. Dans le cas où deux de ces dispositifs sont présents sur la paire de lunettes, selon un mode de réalisation, chaque dispositif utilise une fréquence d'excitation différente, par exemple l'un des dispositif utilise une fréquence d'excitation de l'ordre de 5 kHz, les variations de tension seront alors plus influencées par les variation de capacitance de la peau, l'autre utilise une fréquence d'excitation de l'ordre de 50 kHz, les variations de tension seront alors plus influencées par la résistance électrique de la peau.

Selon la ou les situations à risque dont la détection est visée par le système objet de l'invention (perte de vigilance, chute, déshydratation) la détection est réalisée en combinant et en traitant tout ou partie des signaux :

- du récepteur (152) infrarouge, excité par l'émetteur infrarouge (151) ;
- de l'accéléromètre triaxial (251) ;
- du capteur barométrique (252) ;
- de la tension aux bornes des électrodes (161, 162)

La fiabilité de la détection est améliorée en ajoutant à ces capteurs :

- une deuxième paire d'émetteur / récepteur infrarouge ;
- un second accéléromètre triaxal, monté symétriquement au premier sur l'autre branche de la paire de lunettes ;
- une seconde paire d'électrode soit sur la même branche soit sur l'autre branche utilisant soit la même fréquence d'excitation soit une fréquence d'excitation différente.

La fiabilité de la détection est encore améliorée, ainsi que la possibilité de discriminer plus finement les situations, en ajoutant aux capteurs précédents :

- un capteur gyroscopique ;
- un compas magnétique.

Ces deux derniers capteurs, seuls ou en combinaison, sont avantageusement intégrés dans un MEMS comprenant l'accéléromètre triaxial.

L'unité de traitement et de calcul est avantageusement répartie entre deux modules (261, 262) respectivement sur les cartes électroniques des branches droite et gauche. À titre d'exemple non limitatif, le module (261) de la branche droite comprend un microprocesseur et des moyens de mémoire, comprenant un programme d'acquisition et de traitement, et assure le traitement des signaux issus des différents capteurs, l'extraction des paramètres pertinents, alors que le module (262) de la branche gauche, assure l'acquisition des signaux des capteurs placés sur cette même branche et leur transmission vers le module de la branche droite, la gestion de l'alimentation et de la charge de la batterie (270) et les communications, sans fil ou filaires vers d'autres dispositifs, notamment vers un téléphone intelligent, un ordinateur, ou un boîtier passerelle de connexion WiFi®.

La paire de lunettes comprend finalement des moyens d'alarme répartis entre les branches, par exemple une diode électroluminescente (282) de couleur et un vibreur (281).

Selon un mode de réalisation un connecteur miniature (non représenté), par exemple de type micro-USB (« *Universal Serial Bus* ») est intégré à l'une des branches et permet l'échange de données avec d'autres dispositifs via un lien filaire et le rechargement de la batterie (270).

L'utilisation d'un nombre réduit de capteurs et d'une électronique fortement intégrée, permettent de réaliser la monture des lunettes du système objet de l'invention avec une masse inférieure à 40 grammes sans les verres, et d'assurer une autonomie d'au moins 8 heures par charge, en fonctionnement.

Avantageusement, les lunettes du système objet de l'invention comportent des branches pliables, afin d'être utilisées, transportées et rangées comme des lunettes de vues classiques, plus particulièrement pour permettre leur rangement dans un étui afin de protéger les verres lorsque l'utilisateur ne porte pas lesdites lunettes. La répartition des modules électroniques entre la branche droite et la branche gauche des lunettes, implique le passage d'un bus de transmission entre les deux branches et le passage de ce bus par les charnières desdites branches.

À cette fin, les lunettes du dispositif objet de l'invention comprennent des charnières spécifiques pour l'articulation des branches, lesquelles charnières assurent le pliage dudit bus de transmission selon un rayon de courbure suffisant pour éviter toute dégradation de celui-ci.

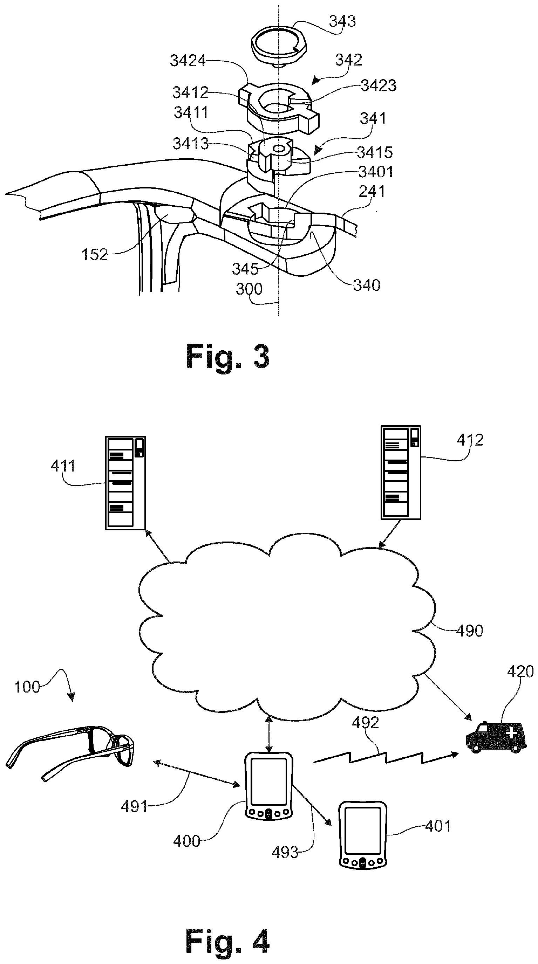

Figure 3, un exemple de réalisation de la charnière de la branche droite des lunettes du système objet de l'invention est représentée dans une position correspondant à la branche dépliée. La branche est supportée par une surface d'appui (340) en épaulement à l'extrémité du cercle, ce qui assure son positionnement vertical précis selon l'axe d'articulation (300). L'articulation est réalisée entre un moyeu (342) lié à la branche et un essieu (341) logé dans la partie fixe de la monture formant la surface d'appui (340). L'essieu (341) comprend deux parties, une première partie (3411) qui vient se loger dans un alésage (3401) de forme complémentaire, réalisé dans la partie fixe, et une deuxième partie (3412), de diamètre inférieur, autour de laquelle s'articule de moyeu (342). La première partie de l'essieu est indexée en rotation dans l'alésage, par exemple, par un moyen de tenon et mortaise et maintenue dans celui-ci, par exemple par clippage ou par collage. Le bus (241) comprend un décrochement (345) de sorte que celui-ci chemine dans la branche et descend dans alésage (3401) recevant l'essieu (341) pour rejoindre le cercle des lunettes. La première partie (3411) de l'essieu comporte à cette fin un fente (3413) pour le passage dudit bus (241). Cette première partie (3411) de l'essieu s'étend sur environ 3/4 d'un cercle pour autoriser le débattement du bus (341) dans l'alésage (3401) lors du pliage et de l'ouverture des branches, ce mouvement représentant un débattement égal ou légèrement supérieur à 90°. Le moyeu (342) monté dans la branche, comporte également une fente (3423), pour le passage du bus (241) dans la partie côté branche dudit bus et avant le décrochement (345) pour sa pénétration dans l'alésage, ladite fente (3423) étant sensiblement diamétralement opposée à la fente (3413) de l'essieu lorsque la branche est dépliée. Ledit moyeu est monté dans un alésage de forme complémentaire pratiqué dans la branche, indexé en rotation dans celle-ci, par exemple par un moyen de tenon et mortaise, et maintenu dans celle-ci, par exemple, par clippage ou collage. Lors du pliage de la branche, la partie du bus (241) entrant dans la fente (3413) de l'essieu ne se déplace pas, seule la partie maintenue dans la fente (3423) du moyeu, jusqu'au décrochement (345) du bus (241) se déplace. La deuxième partie (3412) de l'essieu comprend une zone (3415) de diamètre inférieur au diamètre de guidage en rotation du moyeu, zone sur laquelle glisse le décrochement (345) du bus, lors de la rotation. Ainsi, le rayon de cette zone définit le rayon de courbure imposé au bus (241) lors des manoeuvres de pliage et de dépliage des branches. L'ensemble est maintenu en position par un rivet (343). Avantageusement, un mécanisme d'indexage est inclus dans le tenon de l'essieu réalisant l'arrêt en rotation dudit essieu dans son l'alésage (3401). Le moyeu comporte un tenon (3425) coopérant avec ce mécanisme d'indexage pour indexer la branche en position ouverte et éviter la dégradation du bus (241) par un angle d'ouverture trop important, la position du récepteur infrarouge (152) ne permettant pas de limiter ce déplacement angulaire par une butée de la branche sur le cercle, comme cela est couramment réalisé pour une paire de lunettes classique.

Selon un exemple de réalisation, la paire de lunettes du système objet de l'invention fonctionne de manière totalement autonome, en déterminant les paramètres relatifs à une situation à risque et en générant des alertes vers ses moyens propres d'alerte en interprétant ces paramètres à partir du micro-programme contenu dans l'unité de traitement et de calcul.

Figure 4, selon un autre mode de réalisation du système objet de l'invention, la paire de lunettes (100) est dite connectée, et apte à communiquer soit en permanence, soit périodiquement avec un autre objet (400) par une liaison (491), ladite liaison étant sans fil de type Bluetooth® low energy, ou encore Zigbee®, ou par des moyens filaires.

Selon des exemples de réalisation, ledit objet (400) est un téléphone intelligent, un micro-ordinateur ou un boîtier passerelle de connexion WiFi®. Cet objet est à son tour connecté à un ou plusieurs réseaux, et à d'autres objets (401) ou serveurs (411, 412), par exemple via internet (490), un réseau de téléphonie (492) ou une liaison sans fil de proximité (493) de type Bluetooth®. Ce mode de réalisation permet d'augmenter les fonctionnalité du système.

Ainsi, l'objet connecté (400) est en mesure de récupérer des mises à jour du micro-programme compris dans l'unité de traitement des lunettes, depuis un serveur de mises à jour (412) et charger cette mise à jour dans l'unité de traitement des lunettes lorsqu'il est appairé avec celle-ci.

Ledit objet connecté comprend avantageusement ses propres moyens de calcul et un programme spécifique permettant une analyse des données collectées dans les moyens de mémoire des lunettes, puis en analysant ces données, d'ajuster leur fonctionnement à l'utilisateur, notamment les seuils de déclenchement des alertes, ou les paramètres de calcul de ces seuils.

Le même programme compris dans l'objet connecté (400) est également en mesure de conduire des tests visant à vérifier le bon fonctionnement des lunettes connectées ou à détecter voire traiter des pannes.

Par exemple, ladite paire de lunettes comportant un couple émetteur-récepteur infrarouge sur chaque cercle, si un fonctionnement anormal ou suspect d'un des couples est détecté, de limiter le calcul de la vigilance ou l'estimation du degré d'hydratation de la personne sur les signaux du couple fonctionnant correctement.

Ledit objet connecté (400) est également susceptible, selon des modes de réalisation particuliers, de transmettre des alertes, via différents modes de connexion, tels que internet, réseau de proximité ou réseau de téléphonie cellulaire, à d'autres acteurs. Par exemple, en cas de détection de chute grave, de transmettre une alerte à un centre de secours (420), en transmettant également les coordonnées de localisation de la personne.

Selon un autre exemple de mise en oeuvre, l'objet connecté (400) communique une alerte de baisse de vigilance du porteur des lunettes (100) aux téléphones intelligents (401) des personnes se trouvant à proximité. Ainsi, les passagers d'un véhicule conduit par ledit porteur sont avertis de son état et l'encouragent à arrêter la conduite.

Selon encore un autre exemple, cette alerte de vigilance est communiqué, par exemple, via un réseau de téléphonie cellulaire ou un réseau de téléphonie sans fil de type DECT, au superviseur distant d'un conducteur de machines ou d'un équipement industriel.

Selon encore un autre exemple de réalisation, la détection d'un événement tel qu'une baisse de vigilance ou une chute de sévérité critique, sont transmis à un centre d'assistance, via internet ou via un réseau de téléphonie mobile, avantageusement avec la géolocalisation de la paire de lunettes. Cette géolocalisation est, selon des exemples de réalisation, obtenue depuis les lunettes elles-mêmes si celles-ci comportent une puce de géolocalisation, ou à partir d'un téléphone intelligent appairé avec ladite paire de lunettes comprenant sont propre système de géolocalisation (GPS, ou borne téléphonique).

A réception de cette information, le centre d'assistance prend contact avec l'utilisateur par communication vocale ou par texte afin d'évaluer la situation dudit utilisateur, et par exemple, dans le cas d'une baisse de vigilance aide à le maintenir éveillé et le guider vers une aire de repos la plus proche.

Chaque paire de lunettes (100) est associée à un numéro unique d'identification (UUID) et selon un mode de réalisation, est associé, dans l'application présente sur l'objet connecté à des informations relatives au porteur, telles que son âge, des pathologies éventuelles ou des informations mises à jour à partir des acquisitions réalisées depuis ladite paire de lunettes, telles que sa fréquence moyenne de clignement spontané des yeux ou son taux hydrique massique moyen.

Ces informations, combinées aux mesures réalisées, sont transmises périodiquement, par exemple une fois par jour, et de manière anonyme à un serveur (411) collectant l'ensemble de ces données.

Ainsi, progressivement ce serveur dispose d'une large base de données, sur laquelle des études statistiques ou mettant en oeuvre une intelligence artificielle, couramment désignées sous le terme « *Big Data* », sont réalisées et qui permettent de faire évoluer le système et de proposer des mises à jour personnalisées. Ainsi, le système objet de l'invention met en oeuvre un apprentissage, s'améliore, et s'adapte spécifiquement à son utilisateur.

Cette adaptation comprend deux niveaux. Un premier niveau est réalisé au niveau du dispositif lui-même, en mettant en oeuvre ses propres moyens de calcul, et permet d'adapter les conditions d'alerte aux caractéristiques propres de l'utilisateur sans modifier les algorithmes de traitement.

Un second niveau est atteint par l'analyse des populations et permet d'affiner les algorithmes de traitement par catégorie de détection et par phénotype. Ce second niveau est mis en oeuvre dans un serveur (411) distant.

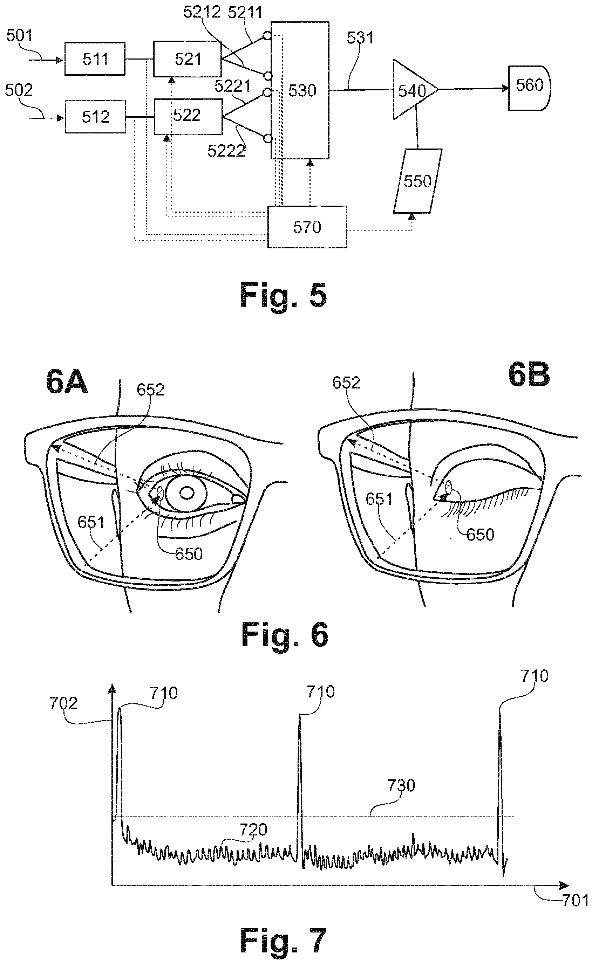

Figure 5, selon un exemple de réalisation la génération d'une alarme liée à une situation à risque donnée, prend en compte l'acquisition de signaux (501, 502) issus d'un ou plusieurs capteurs.

Le signal issu de chaque capteur fait l'objet d'une étape (511, 512) de filtrage spécifique à chaque type de capteur afin d'en éliminer le bruits et les influences non pertinentes. Au cours d'une étape de traitement (521, 522) une série de paramètres (5211, 5212, 5221, 5222) est extraite de chaque signal. Ces paramètres sont combinés au cours d'une étape de calcul (530) afin de définir un indice composite (531) relatif à l'état surveillé.

Cet indice composite (531) est alors comparé (540) à une valeur (550) stockée en mémoire et s'il diffère de cette valeur d'un niveau significatif , une alarme est générée (560).

Les étapes de traitement (521, 522), de calcul (530) et de comparaison (540, 520) mettent en oeuvre des constante enregistrées dans les moyens de mémoire de l'unité de traitement et de calcul. Plusieurs de ces constantes sont propres au porteur du dispositif. Aussi parallèlement au traitement des alarmes, au cours d'une étape d'apprentissage (570), les signaux et les paramètres calculés lors des étapes de traitement (521, 522) sont analysés et les constantes de traitement et de calcul modifiées dans des proportions autorisées, afin de s'adapter à l'individu porteur des lunettes, ce mécanisme correspondant au premier niveau d'apprentissage et de personnalisation du système objet de l'invention.

Figure 6, à titre d'exemple, la mesure de la vigilance et la génération d'alertes relatives à la baisse de vigilance repose essentiellement sur l'analyse des clignements spontanés de la paupière complétés par la détection des micro-chutes de la tête. La mesure des clignements de la paupière est réalisée à partir des signaux issus du récepteur infrarouge.

Figure 6A, lorsque l'oeil est ouvert, le faisceau de lumière incident (651) généré par l'émetteur infrarouge se réfléchit en une tache lumineuse (650) sur la cornée, le récepteur infrarouge mesure l'intensité du faisceau réfléchi (652).

Figure 6B, lorsque la paupière se ferme, le faisceau incident (651) est réfléchi sur la paupière. La réflectance de la paupière étant différente de celle de la cornée, l'intensité lumineuse du faisceau réfléchi (652) est différente.

Ainsi, l'intensité du signal réfléchi (652) varie en fonction de la proportion de paupière se trouvant dans la tache lumineuse du faisceau incident (651). Si la réflectance de la paupière est supérieure à celle de la cornée, de sorte que plus la paupière est fermée et plus l'intensité du signal réfléchi (652), mesurée par le récepteur infrarouge, augmente.

La figure 7, montre un exemple de l'intensité (702) du signal reçu par le récepteur infrarouge en fonction du temps (701). Chaque pic reflète une fermeture plus ou moins complète de la paupière. Ce relevé permet de distinguer des mouvements palpébraux correspondant à des clignements volontaires, correspondant à des pics d'intensité plus élevée, et de plus nombreux pics, de plus faible intensité, correspondant à des clignements spontanés.

Les clignements spontanés sont des mouvements rapides de la paupière dont la personne n'a pas conscience et dont le rôle physiologique est d'éviter la dessiccation de la surface de l'oeil en assurant la collecte et l'excrétion des larmes et l'étalement du film lacrymal. Ces mouvements se produisent selon une fréquence de l'ordre de 20 clignements par minute, variable selon les individus. La fréquence et la vitesse de ces clignement est influencée par des facteurs tels que le stress émotionnel, la fatigue ou la consommation de substances psychotropes, ils constituent par conséquent des indicateurs adaptés à la mesure de la vigilance.

Ainsi, dans l'analyse de la vigilance, seuls sont considérés les pics d'une intensité inférieure à une intensité seuil (730). Ce seuil est déterminé pour un individu donné, lors du réglage et du calibrage des lunettes.

Selon un exemple de réalisation les clignements volontaires ou clin d'oeil, sont utilisables pour piloter des fonctions, notamment de l'objet connecté aux lunettes, dans ce cas, seuls sont considérés les pics d'une intensité supérieure à l'intensité seuil (730) et d'une durée supérieure à une durée définie.

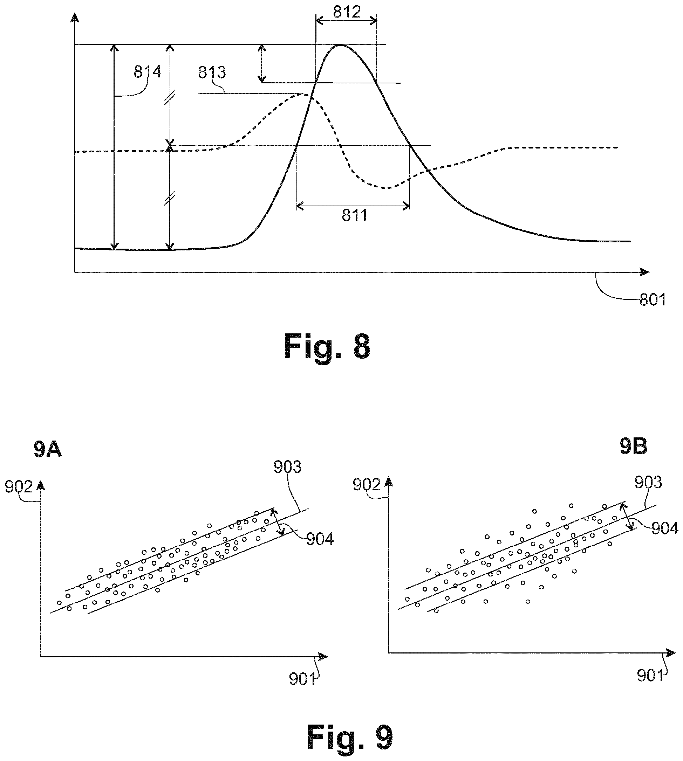

Figure 8, à l'échelle d'un clignement spontané l'analyse du signal (802) issu du récepteur infrarouge et de sa dérivée temporelle (803) en fonction du temps, permet de définir plusieurs paramètres tels que :

- la durée (811) du clignement mesurée par la largeur du pic à mi-hauteur ou plus précisément à la moitié de l'intensité maximale ;
- la durée de fermeture à plus de 80 % de la paupière (812);
- la vitesse maximale de fermeture (813)

L'analyse d'une série de pics sur un temps d'acquisition donné permet de mesurer :

- la fréquence des clignements spontanés ou plus précisément le nombre de clignements spontanés sur un temps donné ;
- la proportion de temps passé avec la paupière fermée à plus de 80 %.

Le début d'un pic est aisément détecté sur la dérivée temporelle du signal. L'opération de dérivation est cependant sensible au bruit présent dans le signal.

À cette fin, le signal issu du récepteur infrarouge, est d'abord filtré de sorte à en éliminer l'influence de l'éclairage ambiant que celui-ci soit naturel ou artificiel. La partie du spectre de cet éclairage se situant dans la plage de mesure du récepteur infrarouge influence la réponse du capteur en ajoutant du bruit et des fréquences supplémentaires.

L'influence de l'éclairage ambiant est ainsi éliminée du signal en appliquant à celui-ci un lissage polynomial à fenêtre glissante ou filtre de Savitsky-Golay, suivi d'un filtrage du signal ainsi lissé par un filtre passe bande, d'une largeur de 10 Hz, de type Butterworth, centré sur la fréquence moyenne de clignement spontané.

Les différents stade de fatigue se caractérisent par une augmentation du temps de fermeture des paupières sur un temps d'interpolation donné, du fait de l'augmentation de la fréquence des clignements spontanés ou de la durée de ces clignements.

Cet effet est saisi par le ratio du cumul du temps passé avec la paupière fermée à plus de 80 % (812) sur la durée d'interpolation, paramètre dénommé dans la suite PERCLOS$_{80}$. Selon un exemple de réalisation, ce paramètre est calculé sur une durée d'interpolation de 20 secondes. Chez un sujet en état d'éveil ce paramètre est inférieur à 3 %. L'augmentation de cette proportion dénote une apparition de la fatigue ou de la baisse de vigilance. Cette proportion de 3 % est indépendante de l'individu de sorte qu'elle permet de définir de manière fiable un état de veille, et de calculer pour cet état de veille, des paramètres plus sensibles à l'état de vigilance mais dépendant de l'individu.

Analysée à l'échelle d'un échantillon de personnes, la baisse de vigilance se traduit par une augmentation de la fréquence des clignements spontanés et par une dispersion de l'intervalle de temps entre deux clignements, notamment par le fait que la durée de certains clignements s'allonge.

Ces paramètres, fréquence de clignement, intervalle de temps moyen entre deux clignements, sur une durée d'interpolation donnée, sont facilement extraits du signal délivré par le récepteur infrarouge. Cependant, si ce paramètre s'avère statistiquement pertinent sur une population d'individus, il est difficile d'en tirer un indicateur précoce de baisse de vigilance sur un individu donné tant les comportements sont variables. Aussi, cet indicateur n'est fiable que pour détecter un état déjà avancé de perte de vigilance.

À cette fin un indicateur est calculé prenant en compte la proportion de clignements présentant une durée de fermeture supérieure à une valeur donnée. Par exemple si cette valeur seuil est fixée à 0,3 secondes, et si, sur 10 pics de clignement successifs cette durée (811) mesurée par la largeur du pic de clignement, plus de 6 clignements ont une durée supérieure à ce seuil, alors l'indicateur prend la valeur scalaire de 0,6 (6/10). Cette durée de 0,3 secondes et cette proportion de 0,6 sont des valeurs hautes, correspondant à une perte de vigilance précédant de peu un endormissement quelque soit l'individu.

Ainsi, de la même manière que le paramètre $PERCLOS_{80}$ permet de définir, lorsqu'il est inférieur à 3 %, de manière fiable un état de veille, ce paramètre nommé $DURATION_{50}$, permet, lorsqu'il atteint un niveau de 0,6, de détecter de manière fiable, un état de perte de vigilance. La détection de ces deux valeurs extrêmes permet de définir, par apprentissage, des seuils relatifs à d'autres paramètres plus sensibles à la vigilance mais plus dépendant de l'individu.

Le paramètre AVR est défini par le rapport entre l'amplitude (814) du pic de clignement et la vitesse maximum de fermeture (813) de la paupière. Ce paramètre est évalué pour chaque pic de clignement spontané sur une durée de mesure donnée, par exemple 3 minutes.

Figure 9A, en reportant les valeurs (902) successives de ce paramètre en fonction du temps pour un sujet en état de veille, celles-ci s'alignent sensiblement sur une droite (903). À partir du nuage de points ainsi acquis un intervalle (904) de prévision est estimé, 90 % des points de mesure se situant autour de cette droite (903) étant compris dans cet intervalle pour un sujet en état d veille. La pente de cette droite et la largeur de l'intervalle de prévision, sont fonctions du sujet, et pour un même sujet sont susceptibles de varier dans le temps.

Figure 9B, lorsque le même sujet, présente des signes de baisse de vigilance, la variance du paramètre AVR augmente, ce qui se traduit par un nombre accru de points se trouvant en dehors de l'intervalle de prévision correspondant au sujet parfaitement éveillé. Ainsi, un indice scalaire de vigilance est obtenu en comptant le nombre de valeurs de l'AVR sortant de l'intervalle de prévision défini pour le sujet éveillé, sur la durée de mesure donnée. Ledit intervalle de prévision doit être calculé pour l'individu. À titre d'exemple la droite (903) et l'intervalle de prévision (904) sont calculés à partir des derniers relevés d'AVR lorsque le sujet est en état de veille confirmé, c'est-à-dire avec un $PERCLOS_{80} < 3$ %, valeurs enregistrées et mises à jour dans les moyens de mémoire de l'unité de traitement et de calcul.

Un paramètre supplémentaire issu de l'accéléromètre permet de détecter les micro-chutes de la tête, leur fréquence ou leur durée, ces paramètres étant caractéristiques d'une perte de vigilance très avancée.

À cette fin, selon un exemple de mise en oeuvre, seule l'accélération selon la direction de la gravité, soit selon l'axe $y$ selon le mode de réalisation représenté figure 2 est utilisée.

En situation normale, l'accéléromètre mesure une accélération de 1g correspondant à la gravité dirigée selon le sens positif de l'axe y.

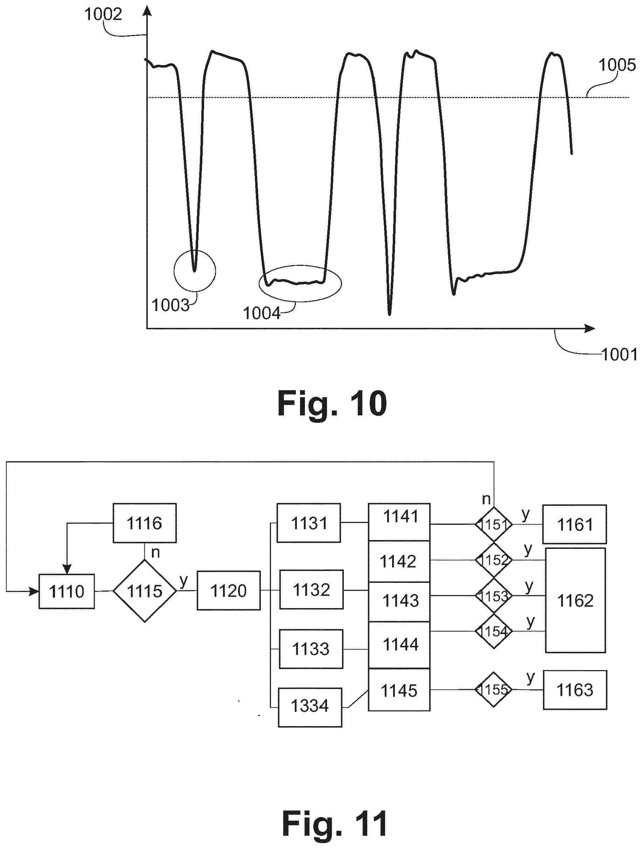

Figure 10, sur un relevé montrant la variation de l'accélération selon l'axe y (1002) en fonction du temps (1002), lors d'une perte de vigilance significative (1003, 1004) engendrant un micro-sommeil, la tête de l'individu tombe vers l'avant selon le sens de l'articulation offrant le moins de résistance. En position d'extension, tête entièrement penchée en avant, l'orientation de l'axe $y$ par rapport à la gravité fait que la projection de celle-ci sur cet axe est inférieure à 1g, puis de nouveau égale à 1g si le sujet redresse la tête. Ainsi, une micro-chute (1003, 1004) de la tête est détectée si l'accélération selon l'axe y prend une valeur inférieure à une valeur seuil (1005).

Le signal est initialement filtré par un filtre passe bas, avec une fréquence de coupure relativement faible, de l'ordre de 2 Hz afin de supprimer du signal les secousses liées à l'activité de l'individu. Ne sont prises en compte que les micro-chutes d'une durée significative, par exemple supérieures à 0,2 seconde. Ainsi, un paramètre scalaire est par exemple déterminé par le nombre de micro-chutes constaté sur une durée de mesure donnée. Un second paramètre scalaire correspond au nombre de micro-chutes d'une durée plus importante (1004), par exemple d'une durée supérieure à 0,5 ou 1 seconde, compté sur le même intervalle de mesure ou sur un intervalle de mesure plus long.

Ces paramètres scalaires:

- $PERCLOS_{80}$, $v_1(t)$
- $DURATION_{50}$ $v_2(t)$
- nombre de points AVR en dehors de l'intervalle de prévision, $v_3(t)$
- nombre de micro-chutes et de micro-chutes longues $v_4(t)$, $v_5(t)$

où, $t$ représente le temps, sont calculés en permanence et sont combinés dans un indice composite qui rend compte de l'état de vigilance du sujet et à partir duquel la décision de générer une alarme est prise.

Le principe de calcul de l'indice composite est similaire quelque soit la situation critique dont la détection est visée, et quelques soient les signaux utilisés, mais utilise différents paramètres en fonction du type de détection visés.

Les paramètres v sont, selon des exemples de réalisation et en fonction de la nature de la détection visée, des scalaires ou des binaires, prenant dans ce dernier cas la valeur 0 ou 1 (ou -1, +1) selon qu'un motif spécifique est détecté ou non dans le signal.

Selon un exemple de mise en oeuvre, les paramètres issus du traitement des signaux des capteurs sont des paramètres scalaires, ou des binaires, fonction du temps et notés $v_1(t) ... v_n(t)$.

Ils sont regroupés dans un vecteur M(t) :

$$M(t) = \begin{bmatrix} v_1(t) \\ \vdots \\ v_2(t) \end{bmatrix}$$

un indice de sévérité relatif à la situation critique *V(t)* est défini par exemple sous la forme :

$$V(t) = A.V(t-1) + B.M(t)$$

*A* et B sont des matrices de coefficients qui sont fonction de l'individu et qui pondèrent l'influence de chacun des paramètres v relativement les uns par rapport aux autres. Selon un exemple simple de mise en oeuvre, à l'instant initial :

$$V(t_0) = V_0 + B.M(t_0)$$

$$B = \begin{bmatrix} \beta_1 & \cdots & \beta_{1n} \\ \vdots & \ddots & \vdots \\ \beta_{n1} & \cdots & \beta_{nm} \end{bmatrix}$$

$$A = [\alpha_1 ... \alpha_n]$$

et *V(t)* est un scalaire.

Selon un exemple de réalisation, les facteurs $\alpha$ et $\beta$ de même que l'équation de combinaison des paramètres pour la détermination de l'indice, évoluent avec l'apprentissage du système, plus particulièrement par l'analyse statistique des données au niveau du serveur (411, figure 4).

En fonction du niveau de l'indice, plusieurs alarmes sont déclenchées. Ainsi, en revenant à la figure 2, et dans l'exemple de la détection et de l'évaluation de la perte de vigilance, un premier niveau d'alarme correspondant par exemple à la détection d'un début de perte de vigilance entraîne l'activation de la diode électroluminescente (282) de manière continue ou clignotante. Un second niveau d'alerte correspondant à une baisse de vigilance plus prononcée, déclenche le vibreur. Un troisième niveau déclenche simultanément la diode et le vibreur, et si le système est configuré de la sorte, adresse un message aux appareils connectés en proximité avec l'objet connecté aux lunettes.

Figure 11, selon un exemple de réalisation, le procédé mettant en oeuvre le système objet de l'invention comprend un première étape (1110) de contrôle du port de la paire de lunettes. Cette étape vise à assurer la cohérence des traitements réalisés mais aussi à placer la paire de lunettes en mode veille si elle n'est pas utilisée afin de réduire sa consommation électrique.

Selon un exemple de réalisation étape de contrôle de port, utilise uniquement les signaux issus du récepteur infrarouge et leur applique un filtrage et un traitement spécifique. Si le test (1115) de port se révèle négatif, la paire de lunettes est mise (1116) en mode veille.

Selon un autre exemple de réalisation, le contrôle de port des lunettes est réalisé à partir des électrodes en contact avec la peau de l'utilisateur. Si les lunettes sont portées, une impédance finie est mesurée entre lesdites électrodes lorsque celles-ci sont alimentées, un circuit électrique étant établi à travers la peau au contact des électrodes. Si les lunettes ne sont pas portées, le circuit électrique est ouvert et l'impédance est infinie.

Lorsque les lunettes sont en mode de veille, un test de port est réalisé de manière régulière, par exemple toutes les minutes, afin de réactiver le mode actif, si le port de lunettes est détecté.

Si le port des lunettes est détecté, l'acquisition des signaux (1120) est lancée, cette acquisition comprend les opérations de filtrage spécifique à chaque signal.

L'acquisition est réalisée à une fréquence comprise en 50 Hz et 150 Hz préférentiellement autour de 70 Hz, qui s'avère être une fréquence permettant de collecter suffisamment de points pour réaliser les traitements visés, tout en limitant la consommation électrique. Les signaux ainsi conditionnés sont adressés à des modules de traitements (1131, 1132, 1133, 1134) qui en extraient les paramètres scalaires ou binaires spécifiques et représentatifs.

Ainsi, selon un exemple de réalisation, un des module de traitement (1131) est par exemple dédié à l'extraction des paramètres issus des signaux du récepteur infrarouge. Un autre module de traitement (1132) est par exemple dédié à l'extraction des paramètres issus des signaux du ou des accéléromètres, un troisième module de traitement (1133) est dédié à l'extraction des paramètres issus du signal du capteur barométrique et un quatrième module (1134) de traitement est dédié au traitement des signaux issus des électrodes.

Les paramètres issus de ces traitements sont utilisés pour être combinés en indices composites, au cours d'une étape de calcul mettant en oeuvre un ou plusieurs modules, par exemple, un module de calcul de la vigilance (1141) utilisant les paramètres définis à partir du signal issus du réflecteur infrarouge et selon un mode de réalisation, au moins un paramètre issu du traitement du signal de l'accéléromètre, un module de calcul (1142) relatif aux chutes utilisant les paramètres issus du traitement des signaux d'accélérométrie et selon un mode de réalisation ceux issus du capteur barométrique, un module de calcul (1143) relatif aux chutes dites molles, utilisant des paramètres calculés à partir des signaux d'accélérométrie et du capteur barométrique, un module de calcul (1144) relatif au rétablissement après chute, utilisant les paramètres issus des traitements du signal d'accélérométrie, du signal du récepteur infrarouge et du signal issu du capteur barométrique, et un module de calcul (1145) relatif à l'état de déshydratation utilisant les signaux issus des électrodes et selon un mode de réalisation les signaux issus du détecteur infrarouge.

Chaque module de calcul définit ainsi un indice de composite relatif à la situation critique dont la détection est visée. Chacun de ces indices composites est comparé à une valeur seuil stockée en mémoire, soit un test (1151) pour la vigilance, un test (1142) pour les chutes, un test (1153) pour les chutes dites molles (1153), un test (1154) pour le rétablissement après la chute et un test (1155) pour l'état de déshydratation.

Si la valeur seuil est dépassée une requête d'alarme est adressée à un module de gestion d'alarme (1161, 1162, 1163). Selon cet exemple de réalisation, deux modules de gestion d'alarme sont utilisés. Un module (1161) de gestion des alarmes relatives à la vigilance qui en fonction de l'écart de l'indice composite avec le seuil active différents moyens d'alarme, tel que décrit plus haut. Un module de gestion d'alarme relative aux chutes, qui en fonction du motif de dépassement de la valeur seuil sur les trois indices relatifs aux chutes (1162) actionne différents moyens d'alarme à partir de tout ou partie des 3 indices de chute, de chute molle, et de rétablissement après une chute, et un module (1163) de gestion des alarmes relatives à la déshydratation.

Si aucun seuil n'est dépassé, l'acquisition et le traitement des signaux continuent sans changement jusqu'à la détection éventuelle d'une condition d'alarme.

Ainsi, à partir d'une même base d'acquisitions réalisées sur un nombre limité de capteurs, jusqu'au modules de traitement (1131, 1132, 1133, 1134), les fonctionnalités du système sont adaptées au besoin en activant ou en chargeant les modules de traitements spécifiques.

Par exemple, si le système objet de l'invention est utilisé par un utilisateur jeune, en bonne santé et n'évoluant pas dans un environnement dangereux, la principale fonctionnalité visée est la surveillance de vigilance, par exemple au cours de la conduite d'un véhicule, et éventuellement l'état de déshydratation.

Dans ce cas, les modules relatifs à la détection et au traitement des chutes (1142, 1143, 1144, 1152, 1153, 1154, 1162) ne sont pas activés, bien que les informations issues de l'accéléromètres restent utilisées, notamment pour la détection des micro-chutes de la tête.

À l'inverse, pour une personne âgée, ne conduisant pas, le principal risque à couvrir est celui de la chute ainsi qu'éventuellement l'état de déshydratation. Dans ce cas les modules déclenchant les alarmes relatives à la vigilance (1141, 1151, 1161) ne sont pas activés, ce qui ne signifie pas pourtant que les informations issues du récepteur infrarouge ne soient pas utilisées, elles le sont notamment dans le module de calcul (1144) de rétablissement après chute et dans le module d'évaluation de l'état de déshydratation.

Finalement pour d'autres cas spécifiques, l'ensemble des modules sont activés.

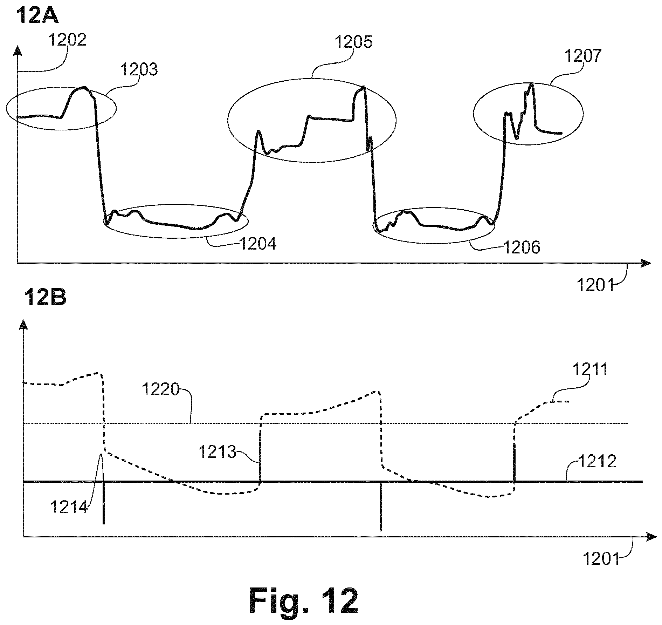

Figure 12, la détection du port des lunettes est obtenue à partir des signaux issus du récepteur infrarouge ou des signaux issus des électrodes.

Figure 12A, en suivant l'évolution de l'intensité (1202) du signal émis par le récepteur en fonction du temps (1201) lors du port et de l'enlèvement successifs des lunettes, lorsque les lunettes sont enlevées (1204, 1206), le faisceau infrarouge émis n'est pas réfléchi par la paupière ou la cornée et l'intensité du signal est faible. En revanche, dès que les lunettes sont portées correctement (1203, 1205, 1207) la réflexion du signal sur la zone oculaire augmente très nettement l'intensité du signal.

Figue 12B, selon un exemple de réalisation, pour détecter le port ou le non-port des lunettes, le signal issu du récepteur infrarouge est fortement lissé (1211) par exemple au moyen d'un filtre à moyenne glissante et pondération exponentielle.

En dérivant le signal filtré, le signal obtenu (1212) permet de détecter facilement les événement de retrait (1214) ou de remise (1213) des lunettes.

Alternativement ou de manière complémentaire, une valeur seuil (1220) est définie de sorte que les lunettes sont portées correctement lorsque l'intensité du signal (1211) ainsi fortement filtré, prend des valeurs supérieures à cette valeur seuil (1220).

Dans le cas d'une détection du port des lunettes à partir de la mesure de l'impédance du circuit électrique entre les deux électrodes, le port est considéré comme incorrect lorsque l'impédance mesurée est supérieures à un seuil défini. Ce seuil dépend de la fréquence du courant d'alimentation, ainsi les variations de capacitance sont plus sensibles dans une gamme de fréquence de l'ordre du kilohertz (soit entre 1 kHz et 10 kHz) alors que les variations de résistance sont plus sensibles dans une gamme de fréquence de l'ordre de la dizaine de kilohertz (soit entre 10 KHz et 100 KHz). Préférentiellement, la détection du port de lunettes utilise une alimentation des électrodes dans la gamme du kilohertz, de sorte à limiter la consommation électrique et mesure ainsi une capacitance.

Bien entendu, selon des modes de réalisation spécifiques, la détection de port combine les données issues du récepteur infrarouge et les données issues de la mesure d'impédance entre les électrodes.

Lorsque les lunettes ne sont pas portées, par exemple parce qu'elles sont tombées suite à une chute, où lorsqu'elles ne sont pas portées correctement, par exemple trop en avant sur le nez, où qu'elles ne reposent pas sur les deux oreilles, le calcul des paramètres, non seulement ceux issus du récepteurs infrarouge mais aussi des autres capteurs est erroné. Aussi le port correct des lunettes est important pour une détection et une évaluation fiable d'une situation à risque.

À cette fin, la mise en veille suite à la détection d'un non-port ou d'un port incorrect est progressive et, selon un exemple de réalisation, commence par l'émission d'une alarme spécifique sur la diode électroluminescente et le vibreur et éventuellement sur l'objet connecté aux lunettes. Puis, lors des tests périodiques suite à la mise en veille, une alarme courte est déclenchée lors de chaque test si le non-port ou le port incorrect sont toujours détecté, par exemple par un déclenchement bref et simultané de la diode et du vibreur, ceci pendant les tests réalisés, par exemple, dans les 15 minutes qui suivent la mise en veille. Au delà de ce délai, le système passe en veille plus profonde et ne génère plus d'alarme.

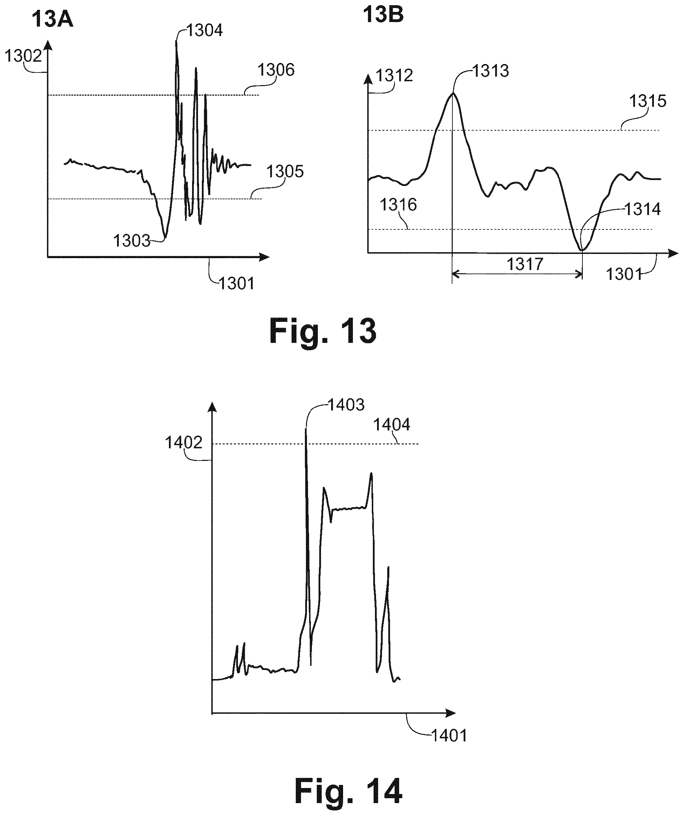

Figure 13, pour la détection des chutes simples, le calcul des paramètres utilise les signaux d'accélérométrie. Les signaux sont initialement filtrés par un filtre passe bas avec une fréquence de coupure relativement basse par exemple 0,1 Hz afin d'en éliminer les phénomènes de vibration correspondant aux activités courantes.

Selon un mode de réalisation, un second filtrage est appliqué sous la forme d'un filtre à médiane glissante, préférentiellement d'ordre 3, ce type de filtrage permet d'éliminer le bruit aléatoire tout en conservant l'acuité des pics.

Figure 13A, compte tenu du positionnement de l'accéléromètre, la gravité étant orientée selon l'axe y positif, un premier effet majeur d'une chute est détecté par une chute de l'accélération mesurée selon l'axe y. Ainsi, selon une version simplifiée du dispositif objet de l'invention, un accéléromètre simple, ne mesurant l'accélération que dans une seule direction, en l'occurrence y, serait suffisant pour une détection de chute. Cependant le phénomène décrit sur l'axe y se détecte également sur la somme des accélérations selon les trois axes, et l'utilisation d'un accéléromètre triaxial permet d'améliorer la détection tout en offrant d'autres avantages, notamment dans la détection de chutes dites molles.

Ainsi, en traçant l'évolution de la somme des accélérations (1302) selon les 3 axes de l'accéléromètre en fonction du temps (1301), un événement de chute se caractérise par l'apparition d'un premier pic (1303) dirigé dans le sens négatif de l'axe (1302) et qui correspond au phénomène de chute libre. Ce premier pic est rapidement suivi d'un deuxième pic (1304) dirigé dans le sens positif de l'axe (1302) et qui correspond à l'impact du corps sur le sol ou tout autre obstacle.

Ainsi, la présence de deux pics inversés consécutifs dans une fenêtre de temps donné et dépassant chacun une valeur seuil (1305, 1306) est un motif spécifique détectable dans la somme des accélérations des capteurs, indicateur d'une chute. À titre d'exemple la valeur de seuil basse est fixée à 0,6g (5,89 ms$^{-2}$) et la valeur de seuil haute (1306), correspondant à l'impact, est fixée à 2g (19,62 ms$^{-2}$). Ces valeurs sont indépendantes du sujet et conduisent à un paramètre v de type binaire selon que ce motif est détecté ou non sur un temps de mesure donné.

Avec ces valeurs seuil, les activités de la vie courante telles que la marche ou la descente d'un escalier ne génèrent pas un motif détectable et donc ne génèrent pas de fausse alarme.

La figure 13B représente l'évolution de la valeur (1312) de la dérivée temporelle du signal issu du capteur barométrique en fonction du temps (1301) au cours d'un épisode de chute suivi d'un relèvement de la personne. Un premier pic (1313) correspond à la chute, c'est-à-dire à la perte d'altitude se traduisant par une augmentation de la pression. Le sujet reste ensuite un certain temps à terre, la pression se stabilise, puis il se relève, ce qui correspond à un second pic (1314) de réduction de la pression ou d'augmentation de l'altitude.

Ces événements se déroulent sur une durée plus importante que la succession des pics d'accélération. Ainsi, un paramètre de caractérisation d'une chute est déterminé sous la forme d'un paramètre binaire, correspondant à la présence d'un pic (1313) dans la dérivée de la pression barométrique dont l'amplitude dépasse un certain seuil.

Une chute d'un mètre en chute libre implique une variation de pression de 12 Pa sur une durée d'environ 0,3 secondes. Une chute est rarement totalement libre de sorte que la valeur seuil est par exemple fixée entre 10 Pas$^{-1}$ et 20 Pas$^{-1}$. Ce paramètre est un paramètre binaire correspondant à la détection ou non d'un tel motif sur un intervalle de temps donné.

Le relèvement après la chute est détectable par la présence d'un second pic (1314) inversé par rapport au premier et dont l'amplitude dépasse une valeur seuil, par exemple comprise entre 5 Pas$^{-1}$ et 10 Pas$^{-1}$. Ainsi un paramètre supplémentaire de caractérisation de la chute est un paramètre binaire indiquant la présence d'un pic de relèvement dans un intervalle de temps donné après le pic de chute sur la dérivée du signal barométrique.

Finalement un paramètre additionnel, de type scalaire, correspondant au temps (1317) séparant le pic de chute (1313) du pic de relèvement (1314) est également utilisable pour caractériser la sévérité de la chute. Ces différents paramètres sont combinés dans un indice composite selon un principe similaire à celui exposé pour la mesure et la détection de la perte de vigilance, lequel indice composite est utilisé pour déclencher différents niveaux d'alarme.

Le principe de traitement exposé ci-avant est efficace pour détecter une chute faisant intervenir un phénomène, même bref, de chute libre, constaté dans le cas d'une chute accidentelle ou dans le cas d'une perte de connaissance brutale.

Cependant, dans certaines circonstances ou pour certaines personnes à risque, la chute peut être causée, par exemple, par une perte progressive de connaissance, conduisant à une chute, dite molle, ne permettant pas de détecter un phénomène de chute libre.

Ce type de chute est cependant critique pour certaines personnes à risque. Si l'épisode de chute libre constaté par le pic (1303) dirigé dans le sens négatif de l'axe (1302) n'est en général pas détectable dans le cas d'une chute molle, le pic d'impact (1304) est cependant détecté bien que d'amplitude moins élevée que dans le cas d'une chute accidentelle.

Il en est de même pour le signal correspondant à la dérivée de la pression barométrique, un pic (1313) correspondant à la perte d'altitude est bien observé mais moins marqué.

Ainsi, une chute molle est caractérisée par la présence d'un pic d'impact détecté sur la somme des accélérations avec une valeur seuil moins élevée que dans le cas d'une chute libre, et de même, par un pic positif dans la dérivée du signal barométrique, détecté pour une valeur seuil moins élevée. Ces deux paramètres sont des paramètres binaires rendant compte de la présence de tels pics dans l'intervalle de mesure.

Toutefois l'utilisation de ces seuls paramètres avec des seuils de détection abaissés présente le risque de la détection de faux négatifs, c'est-à-dire d'interpréter comme une chute une situation de la vie courante, comme s'asseoir sur une chaise ou dans un fauteuil.

Figure 14A, contrairement à une situation de la vie courante comme s'asseoir dans un fauteuil, une chute, même molle, implique une perte de verticalité.

La position des lunettes, sur la tête, est particulièrement avantageuse pour mesurer une telle perte de verticalité. La perte de verticalité est par exemple mesurée par la valeur de l'accélération résultante dans plan perpendiculaire à la gravité, soit les axes *x* et *z* en se référant à la figure 2.

Ainsi le tracé de l'intensité de l'accélération (1402) dans le plan perpendiculaire à la gravité en fonction du temps (1401) au cours d'un épisode de chute molle, fait clairement apparaître un ou plusieurs pics (1403) supérieurs à une valeur seuil (1404) qui sont ainsi détectables et dont la détection sur la durée d'acquisition est capturée dans un paramètre binaire. Ces paramètres sont combinés dans un indice composite afin de détecter une chute molle et générer une alerte.

Cependant, la combinaison de ces paramètres ne permet pas de détecter et de caractériser une chute molle au cours de laquelle la tête reste sensiblement verticale, c'est le cas par exemple dans le cas d'une chute alors que le sujet est adossé contre un mur, ou d'autre situations complexes, et peut conduire à des faux négatifs.

Afin de palier ces insuffisances, l'algorithme complet de détection et de caractérisation des chutes prend en compte des paramètres déterminés dans les instants suivants la chute et qui d'une manière générale attestent du rétablissement, ou non du sujet.

Ces paramètres permettent, entre autre, de réduire le taux de faux négatifs, plus particulièrement en cas de chute molle ou supposée comme telle, en évitant de générer des alarmes voir de prévenir des services d'assistance à la personne dans des situations qui ne le justifient pas.

À titre d'exemple, outre le temps séparant la chute du relèvement éventuel défini figure 13, une chute molle dangereuse se traduit, par exemple, par une perte de connaissance de la personne. Un telle perte de connaissance se manifeste notamment :

- par le maintien de la personne dans une posture non conventionnelle ;
- l'absence d'activité significative de la personne ;
- une activité palpébrale correspondant à une perte de vigilance plus ou moins sévère.

La détermination de la posture de la personne est déterminée par exemple par les valeurs d'accélérométrie sur les différents axes. Lorsque la personne est immobile ou quasi immobile l'accéléromètre n'est soumis qu'à l'action de la pesanteur, qui lorsque la tête est droite se projette selon l'axe y de la figure 2.

Ainsi en mesurant les composantes de l'accélération selon les trois axes, l'orientation de la tête est déterminée. Cette orientation est par exemple définie par un angle en regard de la position théorique verticale. La valeur de cet angle constitue un paramètre scalaire, symptomatique de la posture et de l'activité de la personne après la chute.

L'activité de la personne est, par exemple, également mesurée par l'accéléromètre. Si la personne bouge, des variations d'accélération sont observées. Ainsi l'amplitude de variation des accélérations sur un intervalle de temps donné, mesurée par exemple par la variance du signal d'accélération sur ce temps d'interpolation, est un paramètre scalaire symptomatique de l'activité de la personne.

La mesure de l'activité palpébrale et des paramètres qui en sont déduits a été présentée plus haut.

Évidemment le calcul des paramètres d'activité après la chute n'est pas limité au cas des chutes molles mais est également pertinent en cas de chute accidentelle.

Les différents paramètres ont combinés en un indice composite, selon les principes énoncés précédemment, le niveau duquel indice est utilisé pour décider du déclenchement d'alarmes.

Dans un cas particulier, mais non exceptionnel, la paire de lunettes tombe du visage de la personne ou se trouve en position incorrecte sur celui-ci suite à la chute. Dans ce cas, si des signes de chute sont détectés, les paramètres permettant de caractériser la chute par le comportement du sujet dans les instants suivant la chute ne sont pas mesurables, ou sont mesurés de manière erronée.

Ainsi, selon un mode de mise en oeuvre, suite à la détection d'une chute, la génération d'une alarme fondée sur les paramètres évalués postérieurement à l'incident comprend une vérification du port des lunettes. Si le non port ou un port incorrect est détecté, une alarme est générée et répétée durant un temps défini, tant que les lunettes ne sont pas remises correctement. Si après ce temps défini, malgré l'émission d'alarmes, les lunettes ne sont pas remises, alors il est possible que la personne ne soit pas en mesure de les remettre et une alarme est déclenchée.

L'état de déshydratation d'un individu est déterminé par son taux de mase hydrique, qui donne la quantité d'eau contenu dans son corps en regard de sa masse corporelle.

Lorsque le taux de masse hydrique descend sous une certaine valeur, qui dépend de l'individu, le système objet de l'invention génère une alarme indiquant que la personne se trouve dans des conditions critiques vis-à-vis de la déshydratation.

L'émetteur et le récepteur infrarouge sont utilisables pour évaluer le taux de masse hydrique en mesurant l'absorption de la lumière infrarouge du sang circulant dans les vaisseaux sanguins de la paupière ou de la sclérotique oculaire.

À cette fin, la puissance d'émission de l'émetteur infrarouge est augmentée à intervalles de temps réguliers, par exemple toutes les 10 minutes, de sorte à mesurer l'absorption infrarouge du sang, au moyen de du récepteur infrarouge et ainsi évaluer le niveau de déshydratation de la personne portant les lunettes du système objet de l'invention.

La réflectance de la cornée est aussi fonction de l'hydratation de l'oeuil qui est elle même dépendante de l'état d'hydratation de l'individu.

Ainsi, un indice composite basé sur la combinaison de paramètres calculés à partir de l'absorption infrarouge du sang et de la réflectance de la cornée est calculé pour déterminer la masse hydrique ou l'état de déshydratation de l'individu.

Ces paramètres : absorption infrarouge du sang et réflectance de la cornée, qui sont mesurables par l'émetteur et le réflecteur infrarouge dans un mode de réalisation où les lunettes du système objet de l'invention en sont pourvues, sont fortement dépendant de l'individu et requièrent une procédure de calibrage.

L'impédance de la peau lorsque celle-ci est parcourue par un courant électrique alternatif de faible tension et à haute fréquence est également représentative de l'état d'hydratation d'un individu. Ces variations d'impédance avec le taux de masse hydrique se traduisent par des variations de capacitance et des variations de résistance. L'impédance se situe dans une gamme comprise entre 200 et 500 Ohms, en fonction des caractéristiques du courant (tension et fréquence) mais sont peu dépendantes de l'individu. Ainsi, un seuil d'impédance, indépendant de l'individu, et qui sert par exemple à mesurer un état de déshydratation relativement avancé, est défini et stocké en mémoire.

Avantageusement, l'impédance est mesurée pour différentes fréquences, ainsi dans la gamme du kilohertz ce sont essentiellement des variations de capacitance qui sont détectées, alors que dans la gamme de la dizaine de kilohertz ce sont essentiellement des variations de résistance qui sont détectées.

Ainsi un indice composite calculé à partir des mesures d'impédance réalisées dans les deux gammes de fréquence permet une détection d'un état de déhydratation de manière plus fiable qu'en utilisant une seule mesure.

Finalement, selon un exemple de réalisation, un indice composite, calculé selon les principes exposés précédemment est calculé en combinant les mesures issues du récepteur infrarouge et des mesures d'impédance entre les électrodes. Ainsi, cet indice composite combine des mesures dont le seuil représentatif est dépendant de l'individu, qui nécessitent un calibrage, et des mesures dont le seuil représentatif est indépendant de l'individu.

Avant de pouvoir être utilisées de manière fiable, les lunettes du système objet de l'invention sont préférentiellement adaptées et calibrées pour leur utilisateur.

Cette adaptation comprend une adaptation mécanique, notamment des branches et un calibrage des capteurs, plus particulièrement de l'accéléromètre , de l'émetteur et du récepteur infrarouge selon les capteurs et les modules dont sont équipés les lunettes.

Le calibrage est réalisé par un professionnel, par exemple un opticien, ou par l'utilisateur lui-même, guidé par exemple par une application implantée dans le téléphone intelligent connecté aux lunettes.

Le calibrage de l'accéléromètre, vise à déterminer de manière précise le gain sur chacun des axes et la matrice de rotation de sorte à ce que l'accélération de la pesanteur soit orientée selon l'axe y lorsque l'utilisateur porte les lunettes dans une position considérée comme correspondant à la verticalité. Selon un exemple de réalisation, ce calibrage est réalisé à partir de 3 acquisitions dans des conditions définies.

Selon un première configuration de mesure, les lunettes sont placées parfaitement horizontales en position de port, par exemple dans un montage spécialement adapté à cette fin. Dans ces conditions l'axe *y* est sensé mesurer une accélération de 1g positive. Selon une seconde configuration, les lunettes sont posés parfaitement horizontales, dans une position retournée par rapport à la position de port, par exemple dans un support adapté. Dans cette configuration, l'axe *y* est sensé mesurer une accélération de -1g. Dans une troisième configuration les lunettes sont portées par leur utilisateur en position de tête droite. L'analyse des trois mesures réalisées dans ces conditions permet de calibrer l'accéléromètre.

Le calibrage du récepteur infrarouge permet de fixer les paramètres notamment la valeur seuil dans l'intensité du signal, permettant de différencier les clignements volontaires des clignements spontanés. À cette fin, l'utilisateur porte les lunettes pendant une durée déterminée, par exemple 1 minute, pendant laquelle il effectue un clignement volontaire des deux yeux à des intervalles définis par exemple toutes les 10 secondes. Pendant cette phase d'acquisition, le système ajuste ces paramètres, ceux-ci devant conduire à la détection de 6 pics de clignement volontaires et entre 10 et 20 pics de clignement spontané.

Pour calibrer l'émetteur et du récepteur infrarouge vis-à-vis de la mesure de la déshydratation, le sujet doit être bien hydraté ce qui peut être vérifié par l'intermédiaire de la mesure de l'impédance entre les électrodes, afin de valider les conditions. La puissance d'émission de l'émetteur infrarouge dans le but de la mesure sur la sclérotique oculaire ou sur la paupière est réglée, dans des limites de sécurité, et la valeur réceptionnée sur le récepteur infrarouge est enregistrée en mémoire. La valeur de la réflectance sur la cornée est également réglée mais pour une puissance nominale d'émission de l'émetteur infrarouge, correspondant à la puissance d'émission utilisée pour la détection des mouvements palpébraux et la valeur est également stockée en mémoire.

Ces opérations de calibrage sont avantageusement réalisées de manière périodique.

La description ci-avant et les exemples de réalisation, montrent que l'invention atteint le but visé, à savoir de proposer un système de surveillance de la survenue d'une situation à risque, personnalisé, utilisant un capteur discret esthétique.

**Revendications**

1. Système comprenant une paire de lunettes comprenant des branches articulées et comprenant une pluralité de capteurs et des moyens d'alerte, comprenant au moins 2 capteurs parmi :

   - un accéléromètre triaxial (251) ;
   - un émetteur et un récepteur de lumière infrarouge (151, 152);
   - un capteur barométrique (252) ;
   - une paire d'électrodes (161, 162) alimentées en courant alternatif et des moyens pour mesurer une impédance entre ces deux électrodes ;

   lesdits capteurs étant installés sur les branches ou dans la monture des verres de la paire de lunettes et étant connectés à une unité de traitement et de calcul comprenant :

   - un microprocesseur et des moyens de mémoire ;

   ladite unité de traitement et de calcul comprenant un programme informatique pour l'analyse des données issues des capteurs, et le déclenchement des moyens d'alerte en fonction de l'analyse de ces données.

2. Système selon la revendication 1, comprenant au moins 3 capteurs parmi :

   - un accéléromètre triaxial (251) ;
   - un émetteur et un récepteur de lumière infrarouge (151, 152);
   - un capteur barométrique (252) ;
   - une paire d'électrodes (161, 162) alimentées en courant alternatif et des moyens pour mesurer une impédance entre ces deux électrodes.

3. Système selon la revendication 2, comprenant :

   - un accéléromètre triaxial (251) ;
   - un émetteur et un récepteur de lumière infrarouge (151, 152) ;
   - un capteur barométrique (252) ;
   - une paire d'électrodes (161, 162) alimentées en courant alternatif et des moyens pour mesurer une impédance entre ces deux électrodes ;

4. Système selon l'une des revendications 1 à 3, dans lequel le programme informatique détecte et évalue une situation à risque et son niveau de sévérité à partir d'un premier paramètre issu d'un motif spécifique détecté dans le signal émis par au moins un des capteurs (151, 152, 251, 252, 161, 161), et un second paramètre issu d'un autre motif spécifique détecté dans le signal émis par au moins un des capteurs, les deux paramètres étant combinés dans un indice composite dont le niveau définit le degré de sévérité de la situation et le déclenchement d'un niveau d'alarme.

5. Système selon la revendication 4, dans lequel les deux paramètres sont calculés à partir du signal émis par un même capteur.

6. Système selon la revendication 4, dans lequel les deux paramètres sont calculés à partir de signaux émis par deux capteurs différents.

7. Système selon la revendication 4, dans lequel le premier paramètre est représentatif d'une situation propre à l'utilisateur du dispositif et le second paramètre est représentatif d'un état indépendamment de l'utilisateur.

**8.** Système selon la revendication 1, dans lequel l'unité de traitement et de calcul est portée par la paire de lunettes.

**9.** Système selon la revendication 1, comprenant une unité distante (400, 411, 412) et dans lequel la paire de lunettes (100) comporte des moyens de connexion sans fil aptes à échanger des informations avec ladite unité distante.

**10.** Système selon la revendication 9, dans lequel l'unité distante est un téléphone intelligent.

**11.** Système selon la revendication 1, dans lequel l'unité de traitement et de calcul comprend des moyens de connexion à internet et qui comprend un serveur central (411, 412) apte à échanger des informations avec ladite unité de traitement et de calcul.

**12.** Système selon la revendication 1 dans lequel les au moins 2 capteurs comprennent un accéléromètre triaxal, dans lequel ledit accéléromètre triaxial (251) est placé sensiblement au milieu d'une branche.

**13.** Système selon les revendications 1, dans lequel la paire de lunettes comprend une batterie (270) d'alimentation montée dans une des branches, et dans lequel, les cartes électroniques (211, 212), l'unité de calcul et de traitement (261, 262), la batterie (270) et les capteurs (151, 152, 251, 252, 161, 162) sont positionnés en partie antérieur des branches des lunettes.

**14.** Système selon la revendication 13, dans lequel les branches de la paire de lunettes comprennent une partie postérieure (112) connectée mécaniquement à la partie antérieure (111) desdites branches et dépourvue de moyens électroniques pour être adaptée à la morphologie de l'individu.

Fig. 1

Fig. 2

**Fig. 3**

**Fig. 4**

**Fig. 5**

**Fig. 6**

**Fig. 7**

**Fig. 8**

**Fig. 9**

# Fig. 10

# Fig. 11

EP 3 494 872 A1

**12A**

1202
1203
1205
1207
1204
1206
1201

**12B**

1211
1220
1213
1212
1214
1201

**Fig. 12**

24

Fig. 13

Fig. 14

Europäisches Patentamt
European Patent Office
Office européen des brevets

**RAPPORT DE RECHERCHE EUROPEENNE**

Numéro de la demande

EP 18 21 0290

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | US 2009/105605 A1 (ABREU MARCIO MARC [US]) 23 avril 2009 (2009-04-23) * alinéas [0139], [0600], [0679], [0881] - [0884]; figure 25 * | 1-14 | INV. A61B5/00 |
| A | EP 2 061 026 A1 (SONY CORP [JP]) 20 mai 2009 (2009-05-20) * alinéas [0029], [0030], [0095] - [0099], [0148] - [0158]; figures 1, 3 * | 1-14 | |
| A | US 2001/028309 A1 (TORCH WILLIAM C [US]) 11 octobre 2001 (2001-10-11) * alinéas [0051] - [0077], [0108] - [0125]; figures 6,13 * | 1-14 | |
| A | US 2010/134761 A1 (JOHNS MURRAY [AU] ET AL) 3 juin 2010 (2010-06-03) * alinéas [0023], [0084] - [0095]; figures 8,15 * | 1-14 | |
| X | FR 3 051 656 A1 (ELLCIE HEALTHY [FR]) 1 décembre 2017 (2017-12-01) * pages 5,7; figures 2,4,6 * | 1-14 | DOMAINES TECHNIQUES RECHERCHES (IPC) A61B |
| X,P | WO 2017/207925 A1 (ELLCIE-HEALTHY [FR]) 7 décembre 2017 (2017-12-07) * pages 4,5,23; figure 8 * * pages 26,27 * | 1-14 | |
| X | US 2017/231490 A1 (TOTH LANDY [US] ET AL) 17 août 2017 (2017-08-17) * alinéas [0183] - [0187], [0216] - [0223], [0249] - [0250], [0306], [0315]; figures 5,6 * | 1-14 | |
| A | US 2013/197856 A1 (BARFIELD JAMES R [US] ET AL) 1 août 2013 (2013-08-01) * abrégé * | 4-7 | |

Le présent rapport a été établi pour toutes les revendications

| Lieu de la recherche | Date d'achèvement de la recherche | Examinateur |
|---|---|---|
| Berlin | 24 avril 2019 | Clevorn, Jens |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**     EP 18 21 0290

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

24-04-2019

| Document brevet cité au rapport de recherche | Date de publication | Membre(s) de la famille de brevet(s) | | Date de publication |
|---|---|---|---|---|
| US 2009105605 A1 | 23-04-2009 | US | 2009105605 A1 | 23-04-2009 |
| | | US | 2013124039 A1 | 16-05-2013 |
| | | US | 2015150453 A1 | 04-06-2015 |
| | | US | 2017065183 A1 | 09-03-2017 |
| EP 2061026 A1 | 20-05-2009 | CN | 101506868 A | 12-08-2009 |
| | | EP | 2061026 A1 | 20-05-2009 |
| | | JP | 5228305 B2 | 03-07-2013 |
| | | JP | 2008065169 A | 21-03-2008 |
| | | KR | 20090050069 A | 19-05-2009 |
| | | RU | 2009108342 A | 20-09-2010 |
| | | TW | 200827769 A | 01-07-2008 |
| | | US | 2010013739 A1 | 21-01-2010 |
| | | US | 2013009868 A1 | 10-01-2013 |
| | | US | 2014375559 A1 | 25-12-2014 |
| | | US | 2016124503 A1 | 05-05-2016 |
| | | US | 2017315610 A1 | 02-11-2017 |
| | | WO | 2008029570 A1 | 13-03-2008 |
| US 2001028309 A1 | 11-10-2001 | AUCUN | | |
| US 2010134761 A1 | 03-06-2010 | AT | 481700 T | 15-10-2010 |
| | | BR | PI0614807 A2 | 04-08-2009 |
| | | CA | 2613999 A1 | 15-02-2007 |
| | | CN | 101238497 A | 06-08-2008 |
| | | EP | 1913566 A1 | 23-04-2008 |
| | | IL | 188439 A | 28-04-2011 |
| | | JP | 5133883 B2 | 30-01-2013 |
| | | JP | 2009504205 A | 05-02-2009 |
| | | KR | 20080033191 A | 16-04-2008 |
| | | NZ | 564852 A | 29-01-2010 |
| | | US | 2010134761 A1 | 03-06-2010 |
| | | WO | 2007016739 A1 | 15-02-2007 |
| | | ZA | 200800378 B | 31-12-2008 |
| FR 3051656 A1 | 01-12-2017 | EP | 3463051 A1 | 10-04-2019 |
| | | FR | 3051656 A1 | 01-12-2017 |
| | | US | 2018122208 A1 | 03-05-2018 |
| | | WO | 2017207925 A1 | 07-12-2017 |
| WO 2017207925 A1 | 07-12-2017 | EP | 3463051 A1 | 10-04-2019 |
| | | FR | 3051656 A1 | 01-12-2017 |
| | | US | 2018122208 A1 | 03-05-2018 |
| | | WO | 2017207925 A1 | 07-12-2017 |
| US 2017231490 A1 | 17-08-2017 | AU | 2015302050 A1 | 02-03-2017 |

EPO FORM P0460

Pour tout renseignement concernant cette annexe : voir Journal Officiel de l'Office européen des brevets, No.12/82

page 1 de 2

**ANNEXE AU RAPPORT DE RECHERCHE EUROPEENNE
RELATIF A LA DEMANDE DE BREVET EUROPEEN NO.**

EP 18 21 0290

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

24-04-2019

| Document brevet cité au rapport de recherche | Date de publication | Membre(s) de la famille de brevet(s) | Date de publication |
|---|---|---|---|
| | | CA       2957766 A1 | 18-02-2016 |
| | | EP       3177201 A1 | 14-06-2017 |
| | | SG 11201701018P A | 30-03-2017 |
| | | US    2017231490 A1 | 17-08-2017 |
| | | WO    2016025323 A1 | 18-02-2016 |
| US 2013197856    A1 | 01-08-2013 | AUCUN | |

Pour tout renseignement concernant cette annexe : voir Journal Officiel de l'Office européen des brevets, No.12/82

page 2 de 2

**EP 3 494 872 A1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 17206054 A

- FR 12000